(19) Europäisches Patentamt
European Patent Office
Office européen des brevets

(11) EP 3 207 371 B1

(12) **FASCICULE DE BREVET EUROPEEN**

(45) Date de publication et mention de la délivrance du brevet:
**12.12.2018 Bulletin 2018/50**

(21) Numéro de dépôt: **15804869.4**

(22) Date de dépôt: **16.10.2015**

(51) Int Cl.:
***G01N 33/531*** (2006.01) ***G01N 33/535*** (2006.01)

(86) Numéro de dépôt international:
**PCT/FR2015/052779**

(87) Numéro de publication internationale:
**WO 2016/059351 (21.04.2016 Gazette 2016/16)**

(54) **COMPOSITION POUR DOSAGE IMMUNO-ENZYMATIQUE PAR IMMUNOFLUORESCENCE ET SES UTILISATIONS**

ZUSAMMENSETZUNG FÜR ENZYM-IMMUNOASSAY UNTER VERWENDUNG VON IMMUNFLUORESZENZ UND VERWENDUNG DAVON

COMPOSITION FOR ENZYME IMMUNOASSAY USING IMMUNOFLUORESCENCE AND USES THEREOF

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **17.10.2014 FR 1460003**

(43) Date de publication de la demande:
**23.08.2017 Bulletin 2017/34**

(73) Titulaire: **Biomérieux**
**69280 Marcy-L'Etoile (FR)**

(72) Inventeurs:
- **PAOLICCHI, Aldo**
  **56017 Pisa (IT)**
- **SANESI, Antonio**
  **50126 Firenze (IT)**
- **ROSSI, Veronica Lucia**
  **52100 Arezzo (IT)**
- **IENCO, Andrea**
  **50019 Sesto Fiorentino (IT)**
- **SUSINI, Vanessa**
  **56021 Cascina (IT)**

(74) Mandataire: **Bitaud, Valérie Marie-Odile**
**bioMérieux,**
**Département Propriété Industrielle**
**Chemin de l'Orme**
**69280 Marcy l'Etoile (FR)**

(56) Documents cités:
**US-A- 3 551 295 US-A- 5 854 008**

- **PREETI SALUJA ET AL: "A benzimidazole-based fluorescent sensor for Cuand its complex with a phosphate anion formed through a Cudisplacement approach", TETRAHEDRON LETTERS, PERGAMON, GB, vol. 53, no. 26, 16 avril 2012 (2012-04-16), pages 3292-3295, XP028507944, ISSN: 0040-4039, DOI: 10.1016/J.TETLET.2012.04.062 [extrait le 2012-04-21]**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 3 207 371 B1

## Description

La présente invention concerne une composition pour dosage immuno-enzymatique par immunofluorescence, un kit et un automate d'immuno-analyses contenant une telle composition, ainsi qu'un procédé de détection et/ou quantification *in vitro* d'un analyte d'intérêt par dosage immuno-enzymatique par immunofluorescence associé.

Les méthodes de détection par dosage immunologique sont largement utilisées dans le domaine du diagnostic. Ces méthodes permettent de détecter des analytes dans des échantillons de test sous forme notamment de protéines (antigènes/anticorps), de peptides et d'haptènes comme par exemple les stéroïdes ou les vitamines. Le dosage immunologique, également appelé procédé d'immunodosage, est un procédé largement connu de l'homme du métier impliquant des réactions immunologiques entre l'analyte à détecter et un ou des partenaire(s) de liaison à cet analyte.

Les résultats des dosages immunologiques sont ensuite fournis par un laboratoire à un praticien qui les interprètera pour, par exemple, diagnostiquer un état pathologique ou bien la présence de microorganismes non souhaités, et ensuite prendre les mesures nécessaires, par exemple donner un traitement approprié au patient ou décontaminer un milieu industriel contenant ces microorganismes non souhaités. Il est donc particulièrement important que ces dosages soient à la fois hautement sensibles, en ce sens qu'ils ne donnent pas de résultats faussement négatifs, et hautement spécifiques, en ce sens qu'ils ne donnent pas de résultats faussement positifs.

Les dosages immuno-enzymatiques ou EIA pour « enzyme linked immunoassay » constituent un type de dosage immunologique largement utilisé dans le domaine de l'analyse d'échantillons susceptibles de contenir des analytes d'intérêt. Ces dosages sont couplés à une réaction catalysée par une enzyme en utilisant un substrat enzymatique. Selon le substrat enzymatique choisi, on peut avoir un signal colorimétrique (ELISA pour E*nzyme-Linked Immunosorbent Assay*) (Rassasie, M.J., et al., 1992), un signal de fluorescence (technologie ELFA pour *Enzyme Linked Fluorescent Assay*) ou un signal chemiluminescent (CLIA pour *Chemiluminescence Immuno Assays*) (Stabler T.V., et al., 1991).

Ces méthodes sont basées sur des mesures permettant de quantifier les signaux émis au cours de l'analyse de l'échantillon de test. La quantité de signaux détectée est généralement proportionnelle à la quantité d'analyte à mesurer (par exemple lors d'un dosage en sandwich) ou inversement proportionnelle à la quantité d'analyte à mesurer (par exemple dosage en compétition) US3551295, US5854008, Pretti et al., 2012 (Tetrahedron letters, vol. 53, p. 3292-3295) décrivent des tests de détection de micro-organismes comprenant un composé fluorogénique et un composé fluorogénique éteint. Cependant, les deux composés sont présents de manière séparée et utilisés indépendamment.

La technologie « ELFA » qui permet de quantifier des signaux de fluorescence et d'obtenir des résultats plus sensibles, met en oeuvre une enzyme qui convertit un substrat enzymatique fluorogène en un produit de réaction fluorescent, et éventuellement en un ou plusieurs autres produits de réaction, selon l'équation générale (1) bien connue de l'homme du métier :

$$E + S \rightarrow ES \rightarrow E + S^* \qquad (1)$$

dans laquelle « E » signifie enzyme, « S » signifie substrat enzymatique fluorogène, « ES » correspond au complexe enzyme-substrat et « S* » correspond à un produit de réaction fluorescent issu du substrat enzymatique fluorogène.

Le produit de réaction S* contenu dans le milieu de réaction devient fluorescent lorsqu'il est excité par une lumière d'une longueur d'onde particulière.

En effet, en application du principe de fluorescence, le produit de réaction exposé ou excité par une source lumineuse correspondant à une première longueur d'onde, va émettre à son tour des rayons lumineux ou signaux de fluorescence selon une deuxième longueur d'onde. La première longueur d'onde d'excitation varie généralement dans une zone de 250 à 450 nm. La deuxième longueur d'onde d'émission est, quant à elle, située dans une zone d'émissions situées entre 300 et 600 nm.

Ainsi, la détection des signaux de fluorescence (en unités relatives de fluorescence) associée à un traitement du signal de ces signaux de fluorescence issus du milieu de réaction, lui-même issu de l'échantillon à tester et contenant le produit de réaction, permet de déterminer, par exemple, la présence ou la concentration de l'analyte d'intérêt recherché au sein de l'échantillon.

Toutefois, même si la technologie ELFA est plus sensible que les essais colorimétriques ELISAs, l'homme du métier cherche encore des solutions permettant d'améliorer davantage la détection du signal. En effet, les mesures fluorimétriques sont soumises à des problèmes qui diminuent ou augmentent de façon non spécifique le signal de sortie.

Ainsi, la détection de la fluorescence peut varier en fonction de paramètres tels que le pH, la température, la concentration ionique, le séchage, la présence d'interférences liées à l'échantillon testé ou encore la matrice solide. Ces paramètres influent notamment sur la diffusion de la lumière, sur le bruit de fond, ce qui affecte la sensibilité d'un dosage, notamment par fluorescence (« Selecting the Detection System - Colorimetric, Fluorescent, Luminescent Methods » (Gibbs et al., Elisa Technical Bulletin - No. 5 de 2001).

Compte tenu de ce qui précède, il existe aujourd'hui un besoin de trouver de nouveaux moyens ou des moyens

alternatifs permettant d'augmenter à la fois la spécificité et la sensibilité de la technologie ELFA.

Ainsi, un problème technique que se propose de résoudre la présente invention est de rendre plus efficaces et plus rapides les dosages immuno-enzymatiques par immunofluorescence et, en particulier, d'augmenter la sensibilité, c'est-à-dire d'augmenter la capacité à donner un résultat positif lorsqu'une hypothèse est vérifiée, notamment lorsque l'analyte est présent dans l'échantillon de test en faible quantité.

La solution proposée par l'invention à ce problème a pour premier objet une composition pour dosage immuno-enzymatique par immunofluorescence comprenant (i) un substrat enzymatique fluorogène et (ii) un composé fluorogène éteint formant un composé fluorescent après hydrolyse du substrat enzymatique fluorogène (i).

Plus particulièrement, cette composition selon l'invention comprend ou consiste en :

- d'une part un substrat enzymatique fluorogène permettant, après hydrolyse enzymatique, la formation d'un produit fluorescent et d'un second produit de réaction qui est préférentiellement un anion ; et
- d'autre part un composé fluorogène éteint permettant, après hydrolyse enzymatique dudit substrat enzymatique fluorogène et grâce au second produit de réaction libéré, la formation d'un composé fluorescent issu du composé fluorogène éteint.

En effet, comme indiqué précédemment, il arrive, lors d'une réaction enzymatique, que l'enzyme convertisse le substrat enzymatique fluorogène en un produit de réaction fluorescent, mais entraine également la formation d'un second produit de réaction, considéré généralement comme ne présentant pas d'intérêt particulier. L'équation générale (2) d'une telle réaction enzymatique peut être rédigée comme suit :

$$E + S \rightarrow ES \rightarrow E + S^* + B \qquad (2)$$

dans laquelle « E » signifie enzyme, « S » signifie substrat enzymatique fluorogène, « ES » correspond au complexe enzyme-substrat, « S* » correspond à un produit de réaction fluorescent et « B » correspond à un second produit de réaction, les deux composés « S* » et « B » étant présents dans le milieu réactionnel.

De façon surprenante, la Demanderesse a pu mettre en évidence qu'il était possible d'utiliser le second produit « B » de la réaction enzymatique, non fluorescent, pour le faire réagir avec un composé fluorogène éteint « A ». Après réaction, le produit « B » réagit directement ou indirectement sur le composé fluorogène éteint « A », permettant la génération d'un produit fluorescent « A* » ou « A*-B ».

Par réaction directe du produit « B » sur le composé fluorogène éteint « A », on entend :

- soit que le produit « B » réagit avec le composé fluorogène éteint « A » pour libérer un composé fluorescent « A* » d'une part et un produit de réaction « B' » d'autre part, selon l'équation (3.i) suivante :

$$A + B \rightarrow A^* + B' \qquad (3.i)$$

- soit que le produit « B » se lie spécifiquement au composé fluorogène éteint « A » pour former un complexe fluorescent « A*-B » associant le composé fluorogène éteint « A » au produit « B » selon l'équation (3.ii) suivante :

$$A + B \rightarrow A^*\text{-}B \qquad (3.ii)$$

De façon préférée, le composé fluorogène éteint comprend une partie qui émettra de la fluorescence « A » et un cation libérable « cat », lequel se lit alors « A-cat », et l'équation générale de la réaction chimique directe peut être rédigée comme suit :

$$B + A\text{-cat} \rightarrow \text{cat-}B + A^* \qquad (4)$$

dans laquelle « B » correspond au second produit de réaction détaillé à l'équation générale (2) ci-dessus, « A-cat » signifie composé fluorogène éteint comportant une partie qui pourra émettre de la fluorescence « A* » après libération de son cation « cat », lequel cation « cat » s'associera au produit « B » pour former un produit « cat-B ».

Par réaction indirecte du produit « B » sur le composé fluorogène éteint, on entend que le produit « B » est utilisé dans un ensemble de réactions permettant de transformer le composé fluorogène éteint en composé fluorescent, sans liaison du produit « B » sur ledit composé fluorogène éteint « A ». Le produit « B » se lie alors sur un autre composé

présent dans l'ensemble de réactions, lequel sera alors utilisé dans une étape permettant de rendre fluorescent le composé fluorogène éteint.

Ainsi, de façon innovante, la Demanderesse utilise le produit « B », habituellement considéré comme sans intérêt, issu de la réaction enzymatique détaillée à l'équation (2) ci-dessus, afin d'augmenter le signal fluorescent, en faisant réagir, dans le milieu réactionnel, ledit produit de réaction « B » avec un composé fluorogène éteint. En réutilisant les produits de réaction *a priori* sans intérêt, la Demanderesse arrive ainsi à augmenter considérablement le signal fluorescent.

Cette réutilisation des produits de réaction non fluorescents présente notamment les avantages suivants :

- cela limite l'ajout d'agents additionnels dont la synthèse est compliquée et difficilement reproductible, comme par exemple des dendrimères utilisés pour amplifier le signal, permettant ainsi d'augmenter la sensibilité, la préparation de matières premières et la rapidité des tests ;
- l'augmentation de sensibilité obtenue est réalisée sans changer le temps de réaction ;
- la réaction additionnelle entre le produit de réaction non fluorescent et le composé fluorogène éteint, en particulier en cas de réaction directe entre le produit de réaction « B » et ledit composé fluorogène éteint, est simple, rapide, et nécessite peu d'étapes ;
- elle ne nécessite pas de modification de l'instrument détectant le signal et donc est généralement peu coûteuse ;
- elle permet de détecter des analytes présents en faible concentration dans l'échantillon de test ;
- elle permet d'ajuster les concentrations de composé fluorogène éteint (« A-cat ») pour augmenter la fluorescence des signaux faibles uniquement ;
- la détection du signal de fluorescence du produit de réaction fluorescent (« S* ») et du composé fluorogène éteint « A » ou « A-cat » rendu fluorescent grâce au produit « B » (« A* » ou « A*-B ») se fait dans des gammes de longueurs d'onde situées dans un même intervalle.

L'invention a pour deuxième objet un kit pour dosage immuno-enzymatique par fluorescence comprenant une composition selon l'invention.

Elle a pour troisième objet un automate d'immuno-analyses comprenant une composition selon l'invention.

Elle a pour quatrième objet l'utilisation d'une composition, d'un kit ou d'un automate selon l'invention, pour le dosage immuno-enzymatique par immunofluorescence.

Elle a pour cinquième objet un procédé de détection et/ou quantification *in vitro* d'un analyte par dosage immuno-enzymatique par immunofluorescence de type sandwich dans un échantillon liquide de test susceptible de contenir ledit analyte, comprenant ou consistant en les étapes suivantes de :

- mise en présence d'un partenaire de capture, préalablement fixé ou non fixé sur une surface solide, et dudit échantillon liquide pour la fixation de l'analyte sur le partenaire de capture ;
- ajout d'un partenaire de détection, lequel est couplé directement ou indirectement à une enzyme capable de lyser le substrat enzymatique fluorogène d'une composition de l'invention, pour la fixation sur le complexe partenaire de capture-analyte ;
- mise en présence d'une composition de l'invention et du complexe partenaire de capture-analyte-partenaire de détection pour la formation d'un milieu réactionnel ; et
- détection par immunofluorescence de la présence et/ou la quantité d'analyte en mesurant la fluorescence émise dans le milieu réactionnel.

Elle a pour sixième objet un procédé de détection et/ou quantification *in vitro* d'un analyte par dosage immuno-enzymatique par immunofluorescence dans un échantillon liquide de test susceptible de contenir ledit analyte, comprenant ou consistant en les étapes suivantes de :

- mise en présence d'un partenaire de capture, préalablement fixé ou non fixé sur une surface solide, d'un analogue à l'analyte couplé à une enzyme capable de lyser le substrat enzymatique fluorogène d'une composition de l'invention, et dudit échantillon liquide, lesquels entrent en compétition pour la fixation sur le partenaire de capture ;
- mise en présence d'une composition de l'invention et des complexes partenaires de capture-analyte et partenaire de capture-analogue à l'analyte pour la formation d'un milieu réactionnel ; et
- détection par immunofluorescence de la présence et/ou quantité d'analyte en mesurant la fluorescence émise dans le milieu réactionnel.

Enfin, elle a pour dernier objet un procédé d'amélioration de la sensibilité d'un procédé de détection et/ou quantification *in vitro* par dosage immuno-enzymatique par immunofluorescence d'un analyte d'intérêt dans un échantillon de test, caractérisé en ce qu'il comprend l'utilisation, lors de la mise en oeuvre du dosage, d'une composition selon

l'invention.

L'invention sera mieux comprise à la lecture de la description non limitative qui suit, rédigée au regard des dessins annexés, dans lesquels :

- la figure 1 schématise une réaction connue d'activation d'un substrat enzymatique fluorogène qui est le 4-méthylumbelliferyl phosphate (4-MUP) en 4-méthylumbelliferone (4-MU) et en ion hydrogénophosphate $HPO_4^{2-}$ (Pi pour phosphate inorganique sous toutes ses formes) par l'enzyme phosphatase alcaline (PAL) ;
- la figure 2 schématise une réaction d'activation d'un substrat enzymatique fluorogène qui est ici le 4-méthylumbelliferyl phosphate (4-MUP) optimisée par un composé fluorogène éteint qui est un complexe chimiodétecteur-cation éteint (A-cat) ; le substrat 4-MUP étant converti par l'enzyme phosphatase alcaline (PAL) en 4-méthylumbelliferone (4-MU) et en ion hydrogénophosphate $HPO_4^{2-}$ (Pi pour phosphate inorganique sous toutes ses formes), ledit ion hydrogénophosphate $HPO_4^{2-}$ résultant se liant spécifiquement au cation du complexe chimiodétecteur-cation éteint A-cat pour former d'une part un produit non-fluorescent associant le cation à l'ion phosphate (Pi-cat) et d'autre part un composé fluorescent (A*) ;
- la figure 3 schématise une réaction spécifique d'activation du substrat enzymatique fluorogène 4-méthylumbelliferyl phosphate (4-MUP) optimisée par le complexe chimiodétecteur-cation éteint associant bleu de calcéine et ion Cobalt(« CB-Co ») ; le substrat 4-MUP étant converti par l'enzyme phosphatase alcaline (PAL) en 4-méthylumbelliferone (4-MU) et en ion hydrogénophosphate $HPO_4^{2-}$ (Pi pour phosphate inorganique sous toutes ses formes), ledit ion hydrogénophosphate $HPO_4^{2-}$ résultant se liant spécifiquement au cation du produit chimiodétecteur-cation éteint associant bleu de calcéine et ion Cobalt(« CB-Co ») pour former d'une part un produit non-fluorescent associant le cation à l'ion phosphate (Pi-Co) et d'autre part pour libérer le composé fluorescent bleu de calcéine (CB*)le milieu ;
- la figure 4 illustre que le composé fluorogène éteint, qui est le complexe chimiodétecteur-cation éteint associant bleu de calcéine et ion Cobalt(« CB-Co »), est activé par des concentrations croissantes de phosphate inorganique Pi et permet de former le composé fluorescent bleu de calcéine (CB*) en fonction de ladite concentration en phosphate inorganique Pi [longueur d'onde λ en nm / intensité d'émission en unités arbitraires (a.u.)] ;
- la figure 5 illustre que le spectre d'émission mesuré à l'aide d'un spectrofluorimètre pour les deux solutions suivantes est sensiblement similaire :

    - une solution à pH 9,4 comprenant 6410 nM de bleu de calcéine, 0,6 mM de diéthanolamine (DEA) ; 0,3 mM d'acide éthylène diamine tétraacétique (EDTA) et 0,5 mM de $MgCl_2$ ; et
    - une solution VIDAS® OPT qui comprend 6410 nM de substrat enzymatique fluorogène 4-methylumbelliferyl phosphate (4-MUP) [longueur d'onde λ en nm / intensité d'émission en unités arbitraires (a.u.)]

- la figure 6 illustre le fait que la fluorescence du bleu de calcéine est éteinte après addition d'un ion Cobalt ($Co^{2+}$). [(longueur d'onde λ en nm / intensité d'émission en unités arbitraires (a.u.)].
- la figure 7 schématise une réaction spécifique d'activation du substrat enzymatique fluorogène 4-méthylumbelliferyl phosphate (4-MUP) optimisée par le complexe chimiodétecteur-cation éteint de formule (II) associant un dérivé de benzimidazole à l'ion cuivre ($Cu^{2+}$) :

- 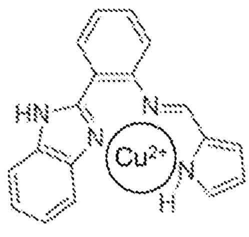 (II)

le substrat 4-MUP étant converti par l'enzyme phosphatase alcaline (PAL) en 4-méthylumbelliferone (4-MU) et en ion hydrogénophosphate $HPO_4^{2-}$ (Pi pour phosphate inorganique sous toutes ses formes), ledit ion hydrogénophosphate $HPO_4^{2-}$ résultant s'associant au cation $Cu^{2+}$ du complexe chimiodétecteur-cation éteint associant un dérivé de benzimidazole à l'ion cuivre ($Cu^{2+}$), pour former d'une part un produit non-fluorescent associant le cation $Cu^{2+}$ à l'ion phosphate (Pi-Cu) et d'autre part pour libérer le dérivé de benzimidazole fluorescent N-[2-(1H-benzo[d]imidazol-2-yl)phényl]-N-[(E)-1-(1H-pyrrol-2-yl)méthylidène]amine de formule (I) suivante:

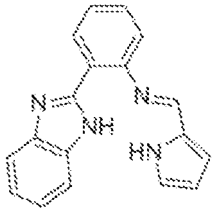

(I);

- la figure 8 schématise les différentes étapes d'un test détaillé à l'exemple 3, permettant notamment de déterminer la fluorescence additionnelle générée par un composé fluorescent selon l'invention ;
- la figure 9 schématise le principe de l'essai PiPer™ [PiPer™ Phosphate Assay kit (Invitrogen™)] dans lequel, en présence de phosphate inorganique, la maltose phosphorylase convertit le maltose en glucose et glucose-1-phosphate. Ensuite, la glucose oxydase convertit le glucose en gluconolactone et $H_2O_2$. Enfin, avec la peroxydase de raifort (HRP), l'$H_2O_2$ réagit avec le réactif non fluorescent Amplex Red pour générer la molécule Résorufine hautement fluorescente ;
- la figure 10 compare la fluorescence moyenne entre d'une part une composition pour dosage immuno-enzymatique par immunofluorescence comprenant uniquement un substrat enzymatique fluorogène qui est le 4-MUP, et d'autre part une composition pour dosage immuno-enzymatique par immunofluorescence selon l'invention, comprenant à la fois un substrat enzymatique fluorogène qui est le 4-methylumbelliferyl phosphate (4-MUP) et un complexe chimiodétecteur-cation éteint associant bleu de calcéine et ion Cobalt(« CB-Co ») ; et
- la figure 11 montre que le complexe chimiodétecteur-cation éteint associant la N-[2-(1H-benzo[d]imidazol-2-yl)phenyl]-N-[(E)-1-(1H-pyrrol-2-yl)methylidene]-amine à l'ion cuivre ($Cu^{2+}$) n'émet effectivement pas de fluorescence, et que la fluorescence est obtenue après introduction de phosphate inorganique Pi dans le milieu [longueur d'onde $\lambda$ en nm / intensité d'émission en unités arbitraires (a.u.)].

L'invention concerne donc une composition pour dosage immuno-enzymatique par immuno fluorescence comprenant (i) un substrat enzymatique fluorogène ainsi que (ii) un composé fluorogène éteint formant un composé fluorescent après hydrolyse enzymatique du substrat (i).

Plus particulièrement, cette composition selon l'invention comprend :

- d'une part (i) un substrat enzymatique fluorogène permettant, après hydrolyse enzymatique, la formation d'un produit fluorescent (appelé premier produit fluorescent) et d'un second produit de réaction qui est préférentiellement un anion ; et
- d'autre part(ii) un composé fluorogène éteint permettant la formation d'un composé fluorescent (appelé second produit fluorescent) grâce notamment à la libération du second produit de réaction qui est préférentiellement un anion issu de l'hydrolyse enzymatique du substrat (i).

Les substrats enzymatiques fluorogènes utiles aux fins de l'invention, que l'on peut appeler substrats primaires dans le cadre de l'invention, sont tous des substrats connus de l'homme du métier donnant, après hydrolyse enzymatique, un produit fluorescent et un second produit de réaction qui est préférentiellement un anion.

Selon un mode particulier de réalisation de l'invention, le substrat enzymatique fluorogène primaire est choisi parmi le 4-méthylumbelliferyl phosphate (4-MUP), le 4-méthylumbelliferyl galactoside (4-MUG), le 4-méthylumbelliferyl sulfate (4-MUS), la fluorescéine di-phosphate (FDP), le 6,8-difluoro-4-méthylumbelliferyl phosphate (DiFMUP), le 9H-(1,3-dichloro-9,9-diméthylacridin-2-one-7-yl) phosphate (DDAO phosphate), le 2'-[2-benzothiazole]-6'-hydroxybenzothiazole phosphate, le 2-naphthyl phosphate et le 2-umbelliferyl phosphate.

Dans ce mode de réalisation particulier, le premier produit fluorescent formé « S* » est la 4-méthylumbelliferone (4-MU), et le second produit de réaction « B » est un anion ou un sucre.

De préférence, le substrat enzymatique fluorogène primaire est choisi parmi le 4-méthylumbelliferyl phosphate (4-MUP), le 4-méthylumbelliferyl sulfate (4-MUS) et le 4-methylumbelliferyl galactoside (4-MUG). Ainsi, de préférence, le second produit de réaction est un anion choisi parmi l'ion hydrogénophosphate $HPO_4^{2-}$ (ou phosphate inorganique noté Pi) et l'ion sulfate $SO_4^{2-}$, ou est un sucre qui est le galactoside.

De préférence encore, le substrat enzymatique fluorogène primaire est choisi parmi le 4-méthylumbelliferyl phosphate (4-MUP) et le 4-méthylumbelliferyl sulfate (4-MUS). Ainsi, de préférence, le second produit de réaction est un ion hydrogénophosphate $HPO_4^{2-}$ (ou phosphate inorganique noté Pi) ou un ion sulfate $SO_4^{2-}$.

Plus préférentiellement encore, le substrat enzymatique fluorogène primaire est le 4-méthylumbelliferyl phosphate (4-MUP) et le second produit de réaction est l'anion hydrogénophosphate $HPO_4^{2-}$.

Les enzymes permettant la formation d'un tel premier produit fluorescent et d'un second produit de réaction sont également connues de l'homme du métier. Parmi les enzymes disponibles pour des dosages immuno-enzymatiques par immunofluorescence, on peut citer notamment la sulfatase, la phosphatase alcaline (PAL), la phosphatase acide, la glucose oxydase (GOx), la glucose-6-phosphate déshydrogénase (G6PD) et la β-galactosidase (β-gal).

Préférentiellement, les enzymes permettant la formation d'un produit fluorescent et d'un second produit de réaction qui est un anion sont choisies parmi la sulfatase, la phosphatase acide et la phosphatase alcaline (PAL).

Selon un mode de réalisation particulier, le substrat enzymatique fluorogène primaire est le 4-méthylumbelliferyl phosphate (4-MUP) qui permet la formation de la 4-méthylumbelliferone (4-MU) et de l'ion hydrogénophosphate $HPO_4^{2-}$ en présence de l'enzyme PAL.

Le premier produit fluorescent issu de la réaction enzymatique est préférentiellement excité à une longueur d'onde comprise entre 250 et 450 nm et émet à une longueur d'onde comprise entre 300 et 600 nm.

Avantageusement, le premier produit fluorescent est excité à une longueur d'onde comprise entre 300 et 400 nm et émet à une longueur d'onde comprise entre 400 et 500 nm.

Plus avantageusement encore, le premier produit fluorescent est excité à une longueur d'onde comprise entre 350 et 380 nm et émet à une longueur d'onde comprise entre 420 et 480 nm.

Par « composé fluorogène éteint », qu'on peut appeler substrat secondaire dans le cadre de l'invention, on entend un composé contenant un groupement qui, lorsqu'il comporte ce groupement fixé, n'est pas capable d'émettre de la fluorescence (*«fluorescence quenching »* en anglais), mais qui émet un signal fluorescent lorsque le groupement est séparé du composé. On peut parler de groupement « quencher » ou « d'extinction ».

Comme exemples de tels composés fluorogènes éteints, on peut citer des dérivés de composés fluorescents classiques pour l'homme du métier, tels que des dérivés de coumarines ou résorufines, lesquels contiennent des groupements empêchant l'émission de fluorescence. A titre d'exemples de tels groupements, on peut citer le groupement $-C(O)-CH_3$ qui, s'il est fixé sur la résorufine, éteint la fluorescence de la résorufine. Un dérivé de résorufine comprenant un tel groupement est le réactif Amplex® Red dont le mécanisme de perte du groupement pour produire de la résorufine fluorescente en présence de phosphate inorganique (réaction indirecte) est décrit dans la notice de $P_i$Per™ Phosphate Assay kit (Invitrogen™) et est schématisé à la figure 9. De façon générale, la perte du groupement « quencher » est réalisée grâce à l'action d'une enzyme, cette enzyme étant la peroxydase de raifort dans l'exemple précédent. De façon générale, ces dérivés de composés fluorescents peuvent être des substrats enzymatiques fluorogènes, différents du substrat enzymatique fluorogène primaire, à la condition qu'ils forment un composé fluorescent (on dit qu'ils sont activés) en présence du second produit de réaction libéré par hydrolyse enzymatique du substrat primaire.

D'autres exemples de composés fluorescents éteints utiles aux fins de l'invention comprennent les chimiodétecteurs sélectifs associés à un cation, lesquels sont éteints sous cette forme associée. Ils sont appelés complexes chimiodétecteur-cation éteints. Comme cela est illustré à la figure 2, ce substrat secondaire de type chimiodétecteur sélectif associé à un cation « A-cat » est apte à être activé par la suite directement par le second produit de réaction généré après hydrolyse enzymatique du substrat enzymatique fluorogène, dans le cas de la figure 2 l'anion « Pi », ce qui va permettre de générer directement un signal additionnel.

L'utilisation de ces derniers composés à titre de composés fluorogènes éteints a pour avantage que le produit de réaction « B », ou « Pi » sur la figure 2, réagit avec le cation du complexe chimiodétecteur-cation éteint et permet :

- la formation d'un complexe associant le produit de réaction au cation (« Pi-cat » à la figure 2) d'une part et
- la formation du composé fluorescent (« A* ») qui permet de produire un second signal fluorescent. Le second signal fluorescent ne nécessite pas l'ajout de composés supplémentaires pour mettre en oeuvre l'ensemble de réactions nécessaires à la formation du composé sous forme fluorescente, comme dans le cas des dérivés de composés fluorescents classiques, ce qui constitue un mode de réalisation particulier de l'invention.

A titre d'exemple non limitatif de chimiodétecteur utilisable, on peut citer les composés fluorescents suivants :

(i) les coumarines hydrophiles, comme décrits par Jung H.S. et al., 2009, Thomas F. et Serratrice G., 1999, et Yao J. et al., 2009, telles que :

- la 7-(diéthylamino)-2-oxo-N-((pyridin-2-yl)-méthyl)-2H-chromène-3-carboxamide :

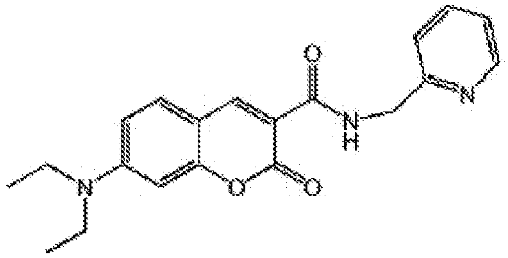

;

- la N-(1,3-dihydroxy-2-(hydroxyméthyl)propan-2-yl)-2-oxo-2H-chromène-3-carboxamide :

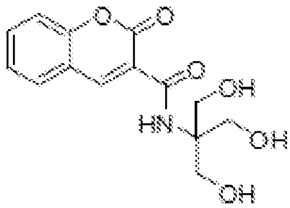

;

- la calcéine :

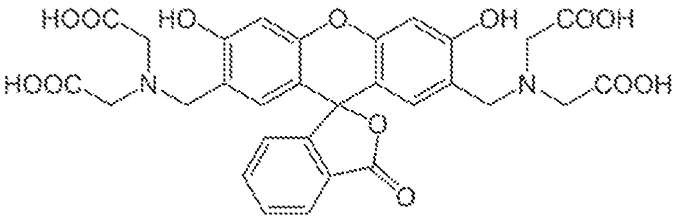

;

et

- le bleu de calcéine :

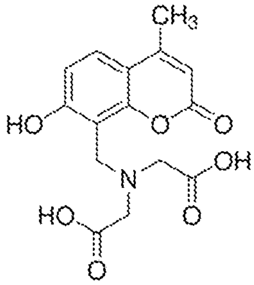

;

(ii) le poly(9-aminofluorène), comme décrit dans Zhang G. et al., 2012 ;

(iii) les dérivés du benzimidazole, comme décrits par Alvaro M. et al., 2001, Henary M.M. et al., 2004 et Saluja P. et al., 2012, tels que :

- la N-[2-(1H-benzo[d]imidazol-2-yl)phényl]-N-[(E)-1-(1H-pyrrol-2-yl)-méthylidène] amine de formule (I) suivante:

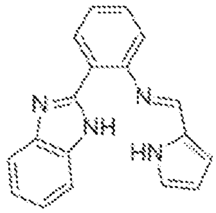

(I) ;

- le macrocycle N, S de dérivé du bis-benzimidazole ;
- l'acide {4-[2-(1H-benzimidazol-2-yl)phényl-sulfamoyl]-phénoxy} acétique :

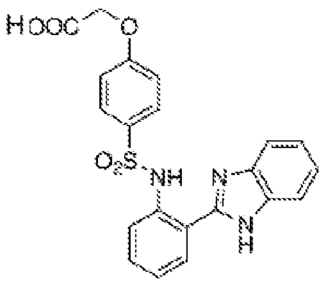

;

- l'acide {4-{2-{4-[(diéthylamino)methyl]-1H-benzimidazol-2-yl}phényl-sulfamoyl}phénoxy} acétique :

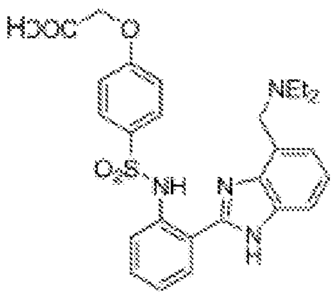

;

- l'acide {4-{2-{4-[(méthylpyridin-2-ylméthylamino)-méthyl]-1H-benzimidazol-2-yl}phénylsulfamoyl}phénoxy} acétique :

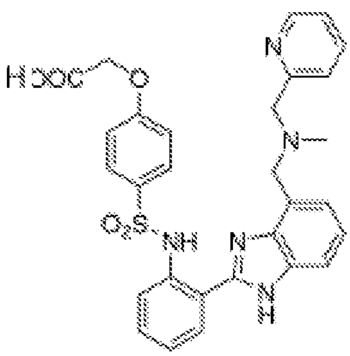

,

et

- l'acide {4-{2-{4-[(bispyridin-2-ylméthylamino)méthyl]-1H-benzimidazol-2-yl}phénylsulfamoyl}phénoxy} acétique :

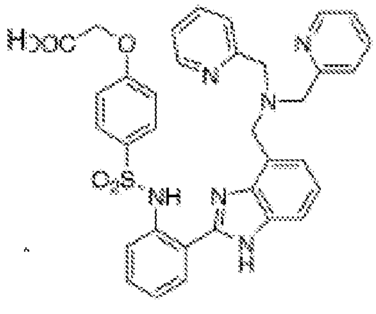

De préférence, le chimiodétecteur utilisé dans la composition selon l'invention est choisi parmi les coumarines hydrophiles et les dérivés du benzimidazole.

De préférence encore, le chimiodétecteur utilisé dans la composition selon l'invention est le bleu de calcéine ou la N-[2-(1H-benzo[d]imidazol-2-yl)phényl]-N-[(E)-1-(1H-pyrrol-2-yl)méthylidène]amine.

De préférence encore, le chimiodétecteur utilisé dans la composition selon l'invention est le bleu de calcéine.

Ledit chimiodétecteur sélectif est associé à un cation pour former un complexe chimiodétecteur-cation éteint.

De préférence, le cation est un métal de transition, de préférence choisi parmi le $Co^{2+}$, le $Cr^{3+}$, le $Cu^{2+}$, le $Fe^{2+}$, le $Fe^{3+}$, le $Mn^{2+}$, le $Ni^{2+}$, le $Hg^{2+}$ et le $Pb^{2+}$.

De préférence encore, le cation utilisé dans la composition selon l'invention est le $Co^{2+}$ ou le $Cu^{2+}$.

De préférence, le complexe chimiodétecteur-cation éteint est choisi parmi :

(i) les coumarines hydrophiles éteintes par un ion métallique telles que les complexes :

- 7-(diéthylamino)-2-oxo-N-((pyridin-2-yl)méthyl)-2H-chromène-3-carboxamide - ion cuivre ($Cu^{2+}$) ;
- N-(1,3-dihydroxy-2-(hydroxyméthyl)propan-2-yl)-2-oxo-2H-chromène-3-carboxamide - ion ferrique ($Fe^{3+}$) ;
- Calcéine - ion ferrique ($Fe^{3+}$) ;
- bleu de calcéine - ion Cobalt ($Co^{2+}$) ;

(ii) les dérivés de benzimidazole soluble dans l'eau éteints par un ion métallique tels que les complexes :

- N-[2-(1H-benzo[d]imidazol-2-yl)phényl]-N-[(E)-1-(1H-pyrrol-2-yl)methylidène] aminé - ion cuivre ($Cu^{2+}$), de formule (II) suivante :

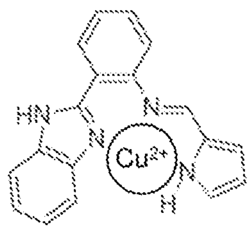

(II) ;

- le macrocycle N, S de dérivé du bis-benzimidazole, éteint par un ion choisi parmi $Cu^{2+}$, $Fe^{2+}$, $Ni^{2+}$, $Co^{2+}$ et $Mn^{2+}$ ;
- l'acide {4-[2-(1H-benzimidazol-2-yl)-phényl-sulfamoyl]phénoxy} acétique, éteint par un ion choisi parmi $Cu2^{+}$, $Fe^{2+}$, $Ni^{2+}$, $Co^{2+}$ et $Mn^{2+}$ ;
- l'acide {4-{2-{4-[(diéthylamino)methyl]-1H-benzimidazol-2-yl}phénylsulfamoyl}phénoxy} acétique, éteint par un ion choisi parmi $Cu^{2+}$, $Fe^{2+}$, $Ni^{2+}$, $Co^{2+}$ et $Mn^{2+}$ ;
- l'acide {4-{2-{4-[(méthylpyridin-2-ylméthylamino)-methyl]-1H-benzimidazol-2-yl}-phénylsulfamoyl}phénoxy} acétique, éteint par un ion choisi parmi $Cu^{2+}$, $Fe^{2+}$, $Ni^{2+}$, $Co^{2+}$ et $Mn^{2+}$ ; et
- l'acide {4-{2-{4-[(bispyridin-2-ylméthylamino)-méthyl]-1H-benzimidazol-2-yl}phénylsulfamoyl}-phénoxy} acétique, éteint par un ion choisi parmi $Cu^{2+}$, $Fe^{2+}$, $Ni^{2+}$, $Co^{2+}$ et $Mn^{2+}$.

Plus préférentiellement encore, le complexe chimiodétecteur-cation éteint est choisi parmi :

- bleu de calcéine - ion Cobalt ($Co^{2+}$) et
- N-[2-(1H-benzo[d]imidazol-2-yl)phényl]-N-[(E)-1-(1H-pyrrol-2-yl)méthylidène]-amine - ion cuivre (Cu2+).

Ledit complexe chimiodétecteur-cation éteint permet, après réaction enzymatique, la formation d'un second produit fluorescent « A* » qui résulte de la séparation du cation du chimiodétecteur fluorescent, le cation se liant avec l'anion par une liaison ionique.

Ce second produit fluorescent est, comme le produit fluorescent « S* », issu de la réaction enzymatique détaillée à l'équation (2), préférentiellement excité à une longueur d'onde comprise entre 250 et 450 nm et émet à une longueur d'onde comprise entre 300 et 600 nm.

Avantageusement, le second produit fluorescent est également excité à une longueur d'onde comprise entre 300 et 400 nm et émet à une longueur d'onde comprise entre 400 et 500 nm.

Plus préférentiellement encore, le premier produit fluorescent et le second produit fluorescent sont excités et émettent à des longueurs d'ondes sensiblement identiques.

Saluja P. et al., 2012 divulgue le composé N-[2-(1H-benzo[d]imidazol-2-yl)phényl]-N-[(E)-1-(1H-pyrrol-2-yl)méthylidène]amine de formule (I) suivante :

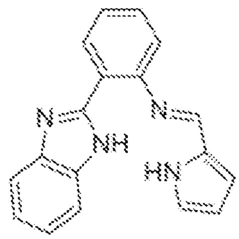

(I)

Ce composé (I) est fluorescent par défaut. Toutefois, lorsque le composé (I) est associé à un cation tel que $Cu^{2+}$, il forme un complexe chimiodétecteur-cation éteint de formule (II) :

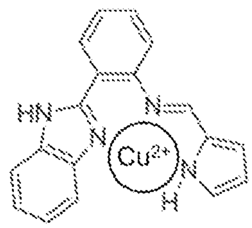

(II)

Enfin, la présence dans le milieu d'un anion tel que le phosphate inorganique va engendrer la liaison du cation $Cu^{2+}$ avec l'ion phosphate, libérant à nouveau le composé (I) fluorescent.

De façon particulièrement avantageuse, la composition selon l'invention associe un substrat enzymatique fluorogène qui est le 4-méthylumbelliferyl phosphate (4-MUP) et un complexe chimiodétecteur-ion éteint qui est le complexe bleu de calcéine - ion Cobalt ($Co^{2+}$).

La composition de l'invention peut également comprendre d'autres composés utiles dans le cadre du dosage biologique tels que des tampons par exemple.

Le substrat enzymatique fluorogène (substrat primaire) et le composé fluorogène éteint (substrat secondaire) présents dans la composition de l'invention peuvent être contenus dans un milieu solide, par exemple une poudre solide, ou dans un milieu liquide.

Par « milieu solide ou liquide », on entend désigner un milieu sous forme solide ou liquide compatible avec l'échantillon à tester susceptible de contenir au moins un analyte représentatif à détecter.

De préférence, le substrat enzymatique fluorogène et le composé fluorogène éteint présents dans la composition de l'invention sont contenus dans un milieu liquide pour dosage immuno-enzymatique.

L'analyte à déterminer peut être une protéine, un peptide ou un haptène, à savoir une réaction qui implique comme partenaire(s) de liaison des antigènes et/ou des anticorps, des récepteurs à l'analyte, etc.

L'échantillon à tester dans le cadre de l'invention peut être de diverses origines, par exemple d'origine alimentaire, environnementale, biologique, vétérinaire, clinique, pharmaceutique ou cosmétique.

Parmi les échantillons d'origine alimentaire, on peut citer, de façon non-exhaustive, un échantillon de produits lactés (yaourts, fromages, etc.), de viande, de poisson, d'oeuf, de fruit, de légume, d'eau, de boisson (lait, jus de fruits,

soda, etc.). Bien évidemment, ces échantillons d'origine alimentaire peuvent aussi provenir de sauces ou de plats plus élaborés ou des matières premières non transformées ou partiellement transformées. Un échantillon alimentaire peut également être issu d'une alimentation destinée aux animaux, telle que des tourteaux, des farines animales. Tous ces échantillons, s'ils ne sont pas liquides, sont préalablement traités pour être sous forme liquide.

Tel qu'indiqué précédemment, l'échantillon peut être d'origine environnementale et peut consister, par exemple, en un prélèvement de surface, d'eau, etc.

L'échantillon peut également consister en un échantillon biologique, d'origine humaine ou animale, pouvant correspondre à des prélèvements de fluide biologique (urine, sang total ou dérivés tels que sérum ou plasma, salive, pus, liquide céphalo-rachidien, etc.), de selles (par exemple diarrhées cholériques), de prélèvements de nez, de gorge, de peau, de plaies, d'organes, de tissus ou de cellules isolées, d'échantillons d'écouvillonnage. Cette liste n'est évidemment pas exhaustive.

D'une manière générale, le terme « échantillon » se réfère à une partie ou à une quantité, plus particulièrement une petite partie ou une petite quantité, prélevée à partir d'une ou plusieurs entités aux fins d'analyse. Cet échantillon peut éventuellement avoir subi un traitement préalable, impliquant par exemple des étapes de mélange, de dilution ou encore de broyage, en particulier si l'entité de départ est à l'état solide.

L'échantillon analysé est, en général, susceptible de - ou suspecté de - contenir au moins un analyte représentatif de la présence de microorganismes ou d'une maladie à détecter, caractériser ou suivre.

L'invention a pour autre objet un kit pour dosage immuno-enzymatique par immunofluorescence d'un analyte susceptible d'être contenu dans un échantillon de test, comprenant une composition telle que définie ci-dessus.

Bien entendu, le terme « immuno » dans « immunodosage » par exemple, n'est pas à considérer dans la présente demande comme indiquant strictement que le partenaire de liaison est un partenaire immunologique, tel qu'un anticorps. En effet, l'homme du métier utilise également largement ce terme lorsque le partenaire de liaison, aussi appelé ligand, n'est pas un partenaire immunologique mais est par exemple un récepteur à l'analyte que l'on veut doser. Ainsi, il est connu de parler du dosage ELISA (« *Enzyme-Linked Immunosorbent Assay* » littéralement « *dosage d'immunoadsorption par enzyme liée »*) pour des dosages qui utilisent des partenaires de liaison non immunologiques, appelés plus largement en anglais « *Ligand Binding Assay* », que l'on pourrait traduire par « *Dosage utilisant la liaison à un ligand* », alors que le terme « immuno » est inclus dans l'acronyme ELISA. Par souci de clarté, la Demanderesse utilisera dans toute la demande le terme « immuno » pour tout dosage utilisant un partenaire de liaison, même quand il n'est pas un partenaire immunologique.

L'invention a pour objet également un automate d'immuno-analyses comprenant une telle composition. Un automate d'immuno-analyses est un automate d'analyses biologiques qui permet de réaliser un certain nombre d'analyses biologiques en un temps limité. Les automates d'immuno-analyses selon l'invention permettent par exemple le dosage des marqueurs cardiaques, le bilan thyroïdien, le bilan anémie, le dosage des marqueurs tumoraux, le bilan fertilité, la réponse virale, la présence de bactéries ou de protéines bactériennes. A titre d'exemples non limitatifs d'automates, on peut citer les automates :

- Access® 2, UniCel™ DxI 600 et UniCel™ DxI 800, commercialisés par la société Beckman Coulter ;
- Advia Centaur™ et Immulite™ commercialisés par la société Siemens ;
- Vitros™ commercialisés par la société Ortho-Clinical-Diagnostics ;
- VIDAS® commercialisés par la Demanderesse ;
- Architect™ commercialisés par la société Abbott Diagnostics ; et
- Elecsys™ commercialisés par la société Roche Diagnostics.

De préférence, l'automate est le VIDAS®, le VIDAS® 3 ou le mini VIDAS®, commercialisés par la Demanderesse.

L'invention concerne également l'utilisation d'une composition, d'un kit ou d'un automate tels que décrits ci-dessus, pour le dosage immuno-enzymatique par immunofluorescence d'un analyte d'intérêt.

L'analyse d'échantillons par dosage immuno-enzymatique met en oeuvre une réaction entre l'analyte d'intérêt et un ou des partenaire(s) de liaison spécifique(s) à l'analyte. Ces partenaires de liaison peuvent être utilisés comme partenaire de capture, comme partenaire de détection, ou comme partenaires de capture et de détection selon le dosage mis en oeuvre.

A titre de partenaire de liaison à l'analyte, on peut citer les anticorps, les fractions d'anticorps, les nanofitines, les récepteurs à cet analyte ou toute autre récepteur moléculaire qui est connu pour avoir une interaction avec l'analyte à rechercher.

Les anticorps partenaires de liaison sont par exemple soit des anticorps polyclonaux, soit des anticorps monoclonaux.

Les anticorps polyclonaux peuvent être obtenus par immunisation d'un animal avec l'analyte ou une partie immunogène de l'analyte, suivie de la récupération des anticorps recherchés sous forme purifiée, par prélèvement du

sérum dudit animal, et séparation desdits anticorps des autres constituants du sérum, notamment par chromatographie d'affinité sur une colonne sur laquelle est fixé un antigène spécifiquement reconnu par les anticorps, notamment l'analyte.

Les anticorps monoclonaux peuvent être obtenus par la technique des hybridomes dont le principe général est rappelé ci-après.

Dans un premier temps, on immunise un animal, généralement une souris avec l'analyte ou une partie immunogène de l'analyte d'intérêt dont les lymphocytes B sont alors capables de produire des anticorps contre cet antigène. Ces lymphocytes producteurs d'anticorps sont ensuite fusionnés avec des cellules myélomateuses "immortelles" (murines dans l'exemple) pour donner lieu à des hybridomes. A partir du mélange hétérogène des cellules ainsi obtenu, on effectue alors une sélection des cellules capables de produire un anticorps particulier et de se multiplier indéfiniment. Chaque hybridome est multiplié sous la forme de clone, chacun conduisant à la production d'un anticorps monoclonal dont les propriétés de reconnaissance vis-à-vis dudit analyte cible pourront être testées par exemple en ELISA, par immunotransfert (Western blot) en une ou deux dimensions, en immunofluorescence, ou à l'aide d'un biocapteur. Les anticorps monoclonaux ainsi sélectionnés sont par la suite purifiés, notamment selon la technique de chromatographie d'affinité décrite ci-dessus.

Les anticorps monoclonaux peuvent être également des anticorps recombinants obtenus par génie génétique, par des techniques bien connues de l'homme du métier.

A titre d'exemple de fragments d'anticorps, on peut citer les fragments Fab, Fab', F(ab')2, ainsi que les chaînes scFv (Single chain variable fragment), dsFv (Double-stranded variable fragment). Ces fragments fonctionnels peuvent notamment être obtenus par génie génétique.

Les nanofitines (nom commercial) sont de petites protéines qui, comme les anticorps, sont capables de se lier à une cible biologique permettant ainsi de la détecter, de la capturer ou tout simplement de la cibler au sein d'un organisme.

Les partenaires de liaison peuvent être spécifiques ou non de l'analyte à détecter. Ils sont dits spécifiques quand ils sont capables de se lier de façon exclusive ou quasi-exclusive à ces analytes. Ils sont dits non spécifiques lorsque la sélectivité de liaison à ces analytes est faible et qu'ils sont alors capables de se lier à d'autres ligands, tels que d'autres protéines ou anticorps. Selon un mode de réalisation préféré, on utilise des partenaires de liaison spécifiques.

La détection de l'analyte est effectuée en mesurant un signal généré après mise en présence du complexe formé entre l'analyte et les partenaires de liaison et de la composition selon l'invention. Elle est mise en oeuvre par une méthode de visualisation d'une fluorescence, par l'intermédiaire d'un marquage, soit du partenaire de détection directement ou indirectement (méthode sandwich), soit de l'analyte lui-même ou d'un ou plusieurs de ses fragments (méthode par compétition).

Par marquage, on entend la fixation d'une enzyme capable de faire générer dans le milieu réactionnel un signal détectable par fluorescence comme par exemple la phosphatase alcaline après hydrolyse d'un substrat enzymatique fluorogène approprié tel que le 4-MUP.

L'invention concerne aussi un procédé de détection et/ou quantification *in vitro* d'un analyte par dosage immuno-enzymatique par immunofluorescence de type sandwich, dans un échantillon liquide de test susceptible de contenir ledit analyte, comprenant les étapes suivantes de :

- mise en présence d'un partenaire de capture, préalablement fixé ou non fixé sur une surface solide, et dudit échantillon liquide pour la fixation de l'analyte sur le partenaire de capture ;
- ajout d'un partenaire de détection, lequel est couplé directement ou indirectement à une enzyme capable de lyser le substrat enzymatique fluorogène d'unecomposition de l'invention, pour la fixation sur le complexe partenaire de capture-analyte ;
- mise en présence d'une composition de l'invention et du complexe partenaire de capture-analyte-partenaire de détection pour la formation d'un milieu réactionnel ; et
- détection par immunofluorescence de la présence et/ou la quantité d'analyte en mesurant la fluorescence émise dans le milieu réactionnel, c'est-à-dire le milieu contenant les premier et second produits de fluorescence tels que décrits précédemment.

Par couplage direct ou indirect de l'enzyme sur le partenaire de détection, on entend que l'enzyme est fixé directement sur le partenaire de détection reconnaissant l'analyte (couplage direct) ou bien l'enzyme est couplé à un partenaire de liaison qui reconnaît le partenaire de détection qui reconnaît lui-même l'analyte (couplage indirect).

Ainsi, dans le cadre du couplage direct, le complexe formé à la fin du dosage sera constitué de :
« Partenaire de capture/analyte/partenaire de détection couplé à l'enzyme ».

Dans le cadre du couplage indirect, le complexe formé à la fin du dosage sera constitué de :
« Partenaire de capture/analyte/partenaire de détection/partenaire de liaison couplé à l'enzyme ». Dans le cadre de ce dernier mode de réalisation, le partenaire de liaison est bien connu de l'homme du métier et peut être par exemple un anticorps anti-IgG (Immunoglobuline) lorsque le partenaire de détection est une IgG reconnaissant l'analyte d'intérêt.

L'invention concerne également un procédé de détection et/ou quantification *in vitro* d'un analyte par dosage

immuno-enzymatique par immunofluorescence de type compétition, dans un échantillon liquide de test susceptible de contenir ledit analyte, comprenant les étapes suivantes de :

- mise en présence d'un partenaire de capture, préalablement fixé ou non fixé sur une surface solide, d'un analogue à l'analyte couplé à une enzyme capable de lyser le substrat enzymatique fluorogène d'une composition de l'invention, et dudit échantillon liquide, lesquels entrent en compétition pour la fixation sur le partenaire de capture ;
- mise en présence d'une composition de l'invention et des complexes partenaire de capture-analyte et partenaire de capture-analogue à l'analyte pour la formation d'un milieu réactionnel ; et
- détection par immunofluorescence de la présence et/ou quantité d'analyte en mesurant la fluorescence émise dans le milieu réactionnel c'est-à-dire le milieu contenant les premier et second produits de fluorescence tels que décrits précédemment.

Hormis l'utilisation de la composition de l'invention, les étapes de ces procédés sont des étapes classiques largement connues de l'homme du métier.
Par ailleurs, les procédés de l'invention peuvent également comprendre une ou plusieurs étapes supplémentaires de rinçage après chaque étape, comme par exemple :

- avant l'ajout du partenaire de détection, une étape de rinçage de façon à éliminer l'analyte non lié au complexe partenaire de capture-analyte ; et
- après l'ajout du partenaire de détection, une étape de rinçage de façon à éliminer le partenaire de détection non lié,

ce qui constitue un autre mode de réalisation de l'invention.
Les étapes de rinçage sont des étapes connues de l'homme du métier. Elles sont mises en oeuvre avec des tampons compatibles avec le milieu réactionnel et la lecture de fluorescence.
Enfin, l'invention concerne un procédé d'amélioration de la sensibilité d'un procédé de détection et/ou quantification *in vitro* par dosage immuno-enzymatique par immunofluorescence d'un analyte à détecter dans un échantillon de test susceptible de contenir l'analyte, caractérisé en ce qu'il comprend l'utilisation, lors de la mise en oeuvre du dosage, d'une composition ou d'un kit selon l'invention.
La présente invention va maintenant être illustrée au moyen des exemples suivants, lesquels ne sont pas donnés de façon limitative.

Exemple 1 : Augmentation de la fluorescence par activation du substrat enzymatique fluorogène 4-methylumbelliferyl phosphate (4-MUP) couplée à la détection sélective du phosphate inorganique Pi.

1. Principes généraux :

*1.1. Réaction standard :*

Comme cela est schématisé à la figure 1, l'étape actuelle de détection par immunofluorescence de la présence d'un analyte dans les immunoessais mis en oeuvre dans l'instrument VIDAS® (bioMérieux) est basée sur l'activation du substrat enzymatique fluorogène 4-méthylumbelliferyl phosphate (4-MUP), contenu dans la dernière cuve (puits 10 ou XA) de la barrette VIDAS®.
Dans la configuration actuelle, lors du dernier mélange de chaque essai mis en oeuvre dans l'instrument VIDAS®, le signal généré par l'enzyme phosphatase alcaline (PAL ou AP) est proportionnel à la quantité d'analyte à doser (méthode sandwich) ou inversement proportionnel à une telle quantité (méthode par compétition). L'enzyme est indirectement fixée aux parois du réceptacle en phase solide (SPR® pour Solid Phase Receptacle) via la formation du complexe partenaire de capture/analyte/partenaire de détection lié à la PAL ou partenaire de capture/analogue de l'analyte lié à la PAL, selon le type d'immunodosage choisi, et réagit avec le substrat enzymatique fluorogène 4-méthylumbelliferyl phosphate (4-MUP) pour générer deux produits :

- la 4-méthylumbelliferone (4-MU) qui est une molécule hautement fluorescente dont la fluorescence est mesurée par la tête optique de l'instrument VIDAS® ; et
- l'ion hydrogénophosphate $HPO_4^{2-}$ (ou Pi pour phosphate inorganique sous toutes ses formes) qui reste en solution sans présenter de rôle particulier.

La figure 1 schématise cette réaction connue.

*1.2. Réaction améliorée :*

La figure 2 schématise une réaction améliorée de détection par immunofluorescence selon l'invention. Cette réaction consiste d'une part en l'activation classique d'un substrat enzymatique fluorogène, qui est ici le 4-méthylumbelliferyl phosphate (4-MUP). Cette réaction est par ailleurs optimisée grâce à un complexe chimiodétecteur-cation éteint (losange « A-cat » schématisé en figure 2).

Comme pour la réaction schématisée à la figure 1, le substrat enzymatique fluorogène primaire 4-MUP est converti par l'enzyme phosphatase alcaline (PAL) en 4-méthylumbelliferone (4-MU) et en ion hydrogénophosphate $HPO_4^{2-}$ (Pi pour phosphate inorganique sous toutes ses formes). Cet ion phosphate (Pi) résultant réagit avec le complexe chimiodétecteur-cation éteint (substrat secondaire) pour libérer dans le milieu d'une part le composé fluorescent « A* » et d'autre part pour former un produit associant l'ion phosphate (Pi) avec le cation « cat »

Ainsi, la méthode proposée ici consiste à ajouter au premier substrat, qui est dans cet exemple le substrat enzymatique fluorogène 4-méthylumbelliferyl phosphate (4-MUP), un substrat secondaire, qui est un complexe chimiodétecteur-cation éteint. Ce substrat secondaire est apte à être activé par la suite par un anion, qui est dans cet exemple le phosphate inorganique Pi, et qui va permettre de générer un signal additionnel (cf. schéma de la figure 2).

Le complexe fluorescent ainsi formé, qui associe le chimiodétecteur sélectif au phosphate inorganique contribue à renforcer le signal global généré par la réaction primaire du 4-methylumbelliferyl phosphate (4-MUP).

2. Mise en oeuvre de la réaction avec le fluorophore « bleu de calcéine » :

Comme cela est illustré à la figure 3, la réaction améliorée détaillée au point 1.2 ci-dessus a été mise en oeuvre par la Demanderesse en utilisant comme substrat secondaire le complexe associant le chimiodétecteur « bleu de calcéine » (CB pour « Calcein Blue ») éteint par le cation Cobalt ($Co^{2+}$)

Le bleu de calcéine ou acide 4-méthylumbelliférone-8-méthyliminodiacétique est un composé dont la formule générale est reprise ci-dessous :

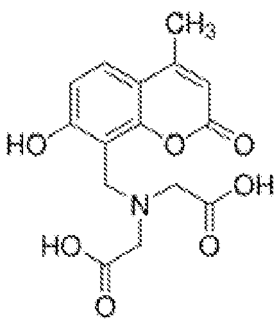

Le bleu de calcéine est une coumarine fluorophore qui présente des caractéristiques semblables à la 4-méthylumbelliférone (4-MU) de formule générale :

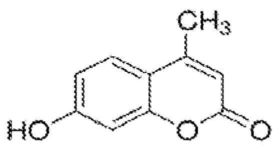

Ceci signifie que le bleu de calcéine est compatible avec les système VIDAS®.

Le complexe chimiodétecteur-cation éteint associant bleu de calcéine et ion Cobalt(« CB-Co ») a été sélectionné comme candidat potentiel pour les raisons suivantes :

- il est sélectif pour le phosphate inorganique Pi ;
- il est, comme cela est illustré à la figure 4, bien activé par des concentrations croissantes de phosphate inorganique Pi. La figure 4 illustre en effet que le complexe chimiodétecteur-cation éteint associant bleu de calcéine et ion Cobalt(« CB-Co ») est activé par des concentrations croissantes de phosphate inorganique Pi et permet la libération de bleu de calcéine fluorescent en fonction de ladite concentration en phosphate inorganique Pi ;
- il est soluble dans l'eau ;
- il est excité à une longueur d'onde de 370 $\pm$ 5 nm et émet à une longueur d'onde de 450 $\pm$ 20 nm.

- il possède, comme cela est illustré à la figure 5, une intensité de fluorescence comparable à la 4-méthylumbelliferone (4-MU). La figure 5 compare le spectre d'émission des deux solutions suivantes comprenant du bleu de calcéine (CB) pour la première et de la 4-méthylumbelliferone (4-MU) pour la seconde :
  - une solution à pH 9,4 comprenant 6410 nM de bleu de calcéine, 0,6 mM de diéthanolamine (DEA) ; 0,3 mM d'acide éthylène diamine tétraacétique ($EDTA-Na_2$) et 0,5 mM de $MgCl_2$ ; et
  - une solution VIDAS® OPT qui comprend 6410 nM (6,4μM) de substrat enzymatique fluorogène 4-méthyl-umbelliferyl phosphate (4-MUP) ;
- la liaison entre le bleu de calcéine et l'ion Cobalt ($Co^{2+}$) est stable à différents pH, y compris à pH 9,2 correspondant au pH du VIDAS XA ;
- le complexe chimiodétecteur-cation associant bleu de calcéine et ion Cobalt(« CB-Co ») est, comme cela est illustré à la figure 6, éteint, c'est-à-dire qu'il n'est pas fluorescent avant la séparation de l'ion Cobalt ($Co^{2+}$).

Par ailleurs, la Demanderesse a pu mettre en évidence que l'intensité de la fluorescence, après séparation de l'ion Cobalt ($Co^{2+}$) restait constante pendant une durée d'au moins 20 minutes.

Exemple 2 : Amélioration de l'émission de fluorescence en utilisant une composition selon l'invention

1. Préparation des solutions à tester

Un tampon pH 8,2 a été préparé avec les composés suivants :

- Tris-Base + HCl (pH final de 8,2)
- [$Mg^{2+}$] 0,7mM
- [$EDTA-Na_2$] 0,03mM
- [$Co^{2+}$] 0,03mM

Trois solutions différentes ont été préparées avec ajout du substrat enzymatique fluorogène 4-méthylumbelliferyl phosphate (4-MUP) et/ou du complexe chimiodétecteur-cation éteint associant bleu de calcéine et ion Cobalt(« CB-Co »). Il s'agit des solutions A, B et C suivantes :

Solution A : 0,64 μM de 4-MUP (forme acide libre)
Solution B : 6,4 μM de CB-Co
Solution C : 0,64 μM de 4-MUP + 6,4 μM de CB-Co

2. Test de stabilité des solutions A et B :

La stabilité des solutions A et B a été testée en contrôlant leur fluorescence au cours du temps, ceci afin de vérifier que l'hydrolyse spontanée du substrat enzymatique fluorogène 4-MUP et le déplacement d'ion Cobalt ($Co^{2+}$) du bleu de calcéine CB sont évités.

Les tableaux 1a. et 1b. ci-dessous donnent les résultats en unités de fluorescence relatives (RFU de l'anglais « Relative Fluorescence Units »), mesurées à l'aide de la tête de lecture scanner de l'instrument VIDAS® 3, obtenues en mettant dans l'instrument trois barrettes remplies de la solution A et trois barrettes remplies de la solution B.

La lecture des résultats est réalisée à l'aide d'un ordinateur portable externe utilisant le logiciel VN Test Suite SW qui pilote la tête scanner de l'instrument VIDAS®.

Tableau 1a. :résultats obtenus pour la solution A (0,64 μM 4-MUP)

| Tris-base + HCl 8,2 [Mg] 0,7 mM [EDTA] 0,03 mM [Co] 0,03 mM | | | | | | | |
|---|---|---|---|---|---|---|---|
| Solution A (4-MUP 0,64 μM) | | | | | | | |
| | t0 (RFU) | t5min (RFU) | t10min (RFU) | t15min (RFU) | t20min (RFU) | t25min (RFU) | t30min (RFU) |
| Barrette 1 | 34 | 32 | 33 | 33 | 32 | 33 | 33 |
| Barrette 2 | 30 | 29 | 29 | 29 | 29 | 30 | 30 |
| Barrette 3 | 33 | 32 | 31 | 31 | 32 | 32 | 32 |

Tableau 1b. résultats obtenus pour la solution B (0,64 μM CB-Co) :

| Tris-base + HCl 8,2 [Mg] 0,7 mM [EDTA] 0,03 mM [Co] 0,03 mM | | | | | | | |
|---|---|---|---|---|---|---|---|
| Solution B (CB-Co 0,64 μM) | | | | | | | |
| | t0 (RFU) | t5min (RFU) | t10min (RFU) | t15min (RFU) | t20min (RFU) | t25min (RFU) | t30min (RFU) |
| Barrette 1 | 34 | 34 | 34 | 34 | 34 | 34 | 33 |
| Barrette 2 | 41 | 43 | 43 | 43 | 43 | 43 | 42 |
| Barrette 3 | 36 | 43 | 44 | 45 | 45 | 44 | 43 |

Les résultats montrent que la fluorescence des compositions reste constante et faible. Ainsi, les deux solutions A et B sont stables dans le temps, ce qui démontre que le substrat enzymatique fluorogène 4-MUP ne s'hydrolyse pas spontanément et que le déplacement d'ion Cobalt ($Co^{2+}$) est évité.

3. Comparaison des fluorescences des solutions A et C :

Après ce test de stabilité, le test suivant a été réalisé, à trois reprises :

Etape 1 : Trois barrettes (puits XA) ont été remplies avec 300 μl de la solution A et trois barrettes ont été remplies avec la solution C. Les barrettes ont été chargées dans l'instrument VIDAS® 3 ;
Etape 2 : La valeur du bruit de fond (BKG pour « background ») est mesurée par la tête scanner de l'instrument VIDAS® au temps t0 pour chaque barrette ;
Etape 3 : L'enzyme phosphatase alcaline (PAL) est ajoutée dans chaque cuvette (10 μl à 500 μg/ml) ;
Etape 4 : La fluorescence de chaque cuvette est mesurée pendant une durée de 15 minutes à des instants déterminés ;
Etape 5 : L'augmentation de la fluorescence est calculée. La valeur de fluorescence relative (RFV pour Relative Fluorescence Value) a été déterminée en soustrayant la valeur du bruit de fond (BKG) de la valeur de la dernière lecture à t=15 minutes.

Une durée de 15 minutes a été choisie afin de s'assurer que le substrat enzymatique fluorogène 4-MUP contenu dans la solution A est complètement activé en 4-MU.

Par ailleurs, la fluorescence obtenue avec la solution A après 15 minutes est homogène avec la fluorescence pour une concentration en 4-méthylumbelliferone (4-MU) de 0,6410 μM obtenue en diluant 1/10 d'une solution VIDAS® OPT (à 6,41 μM) dans le même tampon.

Les résultats bruts du test réalisé trois fois sont repris dans les tableaux 2a. et 2b. ci-dessous :

Tableau 2a. résultats obtenus pour la solution A (0,64 μM 4-MUP)

| Tris-base + HCl 8,2 [Mg] 0,7 mM [EDTA] 0,03 mM [Co] 0,03 mM | | | | | |
|---|---|---|---|---|---|
| 4-MUP 0,64 μM | | | | | |
| | T0 (RFU) BKG | +AP 10 μl 500μg/ml | t 5min (RFU) | t 10min (RFU) | t 15min (RFU) |
| Barrette 1.1 | 45 | 330 | 321 | 318 | 317 |
| Barrette 1.2 | 45 | 302 | 295 | 291 | 291 |
| Barrette 1.3 | 58 | 258 | 251 | 249 | 250 |
| Barrette 2.1 | 46 | 295 | 291 | 289 | 288 |
| Barrette 2.2 | 68 | 297 | 282 | 280 | 280 |
| Barrette 2.3 | 46 | 291 | 284 | 285 | 286 |
| Barrette 3.1 | 45 | 321 | 311 | 309 | 309 |
| Barrette 3.2 | 45 | 338 | 330 | 330 | 333 |
| Barrette 3.3 | 43 | 329 | 321 | 319 | 319 |

Tableau 2b. : résultats obtenus pour la solution C (6,4 μM CB-Co + 0,64 μM 4-MUP)

| Tris-base + HCl 8,2 [Mg] 0,7 mM [EDTA] 0,03 mM [Co] 0,03 mM | | | | | |
|---|---|---|---|---|---|
| CB-Co 6,4 μM + 4-MUP 0,64 μM | | | | | |
| | T0 (RFU) BKG | +AP 10 μl 500μg/ml | t 5min (RFU) | t 10min (RFU) | t 15min (RFU) |
| Barrette 1.1 | 109 | 387 | 463 | 501 | 516 |
| Barrette 1.2 | 107 | 381 | 443 | 489 | 513 |
| Barrette 1.3 | 110 | 386 | 458 | 503 | 523 |
| Barrette 2.1 | 97 | 335 | 404 | 471 | 526 |
| Barrette 2.2 | 95 | 333 | 390 | 454 | 525 |
| Barrette 2.3 | 95 | 324 | 388 | 426 | 477 |
| Barrette 3.1 | 99 | 364 | 460 | 529 | 579 |
| Barrette 3.2 | 96 | 378 | 441 | 489 | 529 |
| Barrette 3.3 | 94 | 369 | 475 | 524 | 527 |

Enfin, le tableau 2c. ci-dessous reprend les valeurs relatives de fluorescence (RFV) pour l'ensemble des tests effectués et les pourcentages d'augmentation de la fluorescence correspondants.

Tableau 2c :

| | **Solution A (4-MUP)** | **Solution C (4-MUP + CB-Co)** | **Augmentation (%)** |
|---|---|---|---|
| | **t 15 min - BKG** | | |
| | **RFV** | **RFV** | |
| Barrette 1.1 | 272 | 407 | |
| Barrette 1.2 | 246 | 406 | |
| Barrette 1.3 | 192 | 413 | |
| **RFV moyen test 1** | **237** | **409** | **72%** |
| Barrette 2.1 | 242 | 429 | |
| Barrette 2.2 | 212 | 430 | |
| Barrette 2.3 | 240 | 382 | |
| **RFV moyen test 2** | **231** | **414** | **79%** |
| Barrette 3.1 | 264 | 480 | |
| Barrette 3.2 | 288 | 433 | |
| Barrette 3.3 | 276 | 433 | |
| **RFV moyen test 3** | **276** | **449** | **62%** |

Comme cela est détaillé au tableau 2c. ci-dessus, la fluorescence moyenne augmente d'environ 70% entre la solution A et la solution C contenant l'association du substrat enzymatique fluorogène 4-MUP et du complexe chimiodétecteur-cation éteint CB-Co selon l'invention.

Ainsi l'ajout d'un chimiodétecteur-cation éteint permet d'augmenter le signal fluorescent de façon significative.

Exemple 3 : Utilisation de différentes concentrations de substrat primaire dans une composition selon l'invention :

Tests préliminaires avec un complexe chimiodétecteur-cation éteint associant bleu de calcéine et ion Cobalt(« CB-Co ») :

La Demanderesse a souhaité évaluer l'augmentation de la fluorescence par ajout de complexe chimiodétecteur-cation

éteint CB-Co dans une solution comprenant différentes concentrations de substrat enzymatique fluorogène 4-MUP, par rapport à une solution comprenant uniquement le substrat enzymatique fluorogène 4-MUP, et ce à ces mêmes concentrations.

1. Préparation des solutions à tester

Un tampon pH 8,2 a été préparé comme décrit dans l'exemple 2 ci-dessus.

Trois concentrations différentes de 4-MUP ont été considérées, il s'agit des concentrations suivantes :

(1) 0,64 μM
(2) 0,43 μM
(3) 0,32 μM.

Pour chaque concentration, trois solutions différentes ont été préparées :

*(1) Concentration 0,64μM :*

- Solution A1 : 0,64 μM de 4-MUP (forme acide libre)
- Solution B1 : 6,4 μM de CB-Co
- Solution C1 : 0,64 μM de 4-MUP + 6,4 μM de CB-Co

*(2) Concentration 0,43μM :*

- Solution A2 : 0,43 μM de 4-MUP (forme acide libre)
- Solution B2 : 6,4 μM de CB-Co
- Solution C2 : 0,43 μM de 4-MUP + 6,4 μM de CB-Co

*(3) Concentration 0,32μM :*

- Solution A3 : 0,32 μM de 4-MUP (forme acide libre)
- Solution B3 : 6,4 μM de CB-Co
- Solution C3 : 0,32 μM de 4-MUP + 6,4 μM de CB-Co.

2. Section expérimentale:

Chaque expérience a été effectuée en triple exemplaire et répété trois fois, pour chaque concentration de 4-MUP testée. Le tableau 3 ci-dessous reprend le schéma du plan de chargement du VIDAS™ pour une concentration de 0,64 μM de 4-MUP :

Tableau 3

| | | | |
|---|---|---|---|
| *Echantillons* | *Section A* | | |
| | *Emplacement 1* | *Emplacement 2* | *Emplacement 3* |
| | *CB-Co 6.4μM* | *vide* | *vide* |
| | *Section B* | | |
| | *Emplacement 1* | *Emplacement 2* | *Emplacement 3* |
| | *4-MUP 0.64 μM* | *4-MUP 0.64 μM* | *4-MUP 0.64 μM* |
| | *Section C* | | |
| | *Emplacement 1* | *Emplacement 2* | *Emplacement 3* |
| | *4-MUP 0.64 μM + CB-Co 6.4μM* | *4-MUP 0.64 μM + CB-Co 6.4μM* | *4-MUP 0.64 μM + CB-Co 6.4μM* |

Les solutions B1, B2 et B3 constituent un contrôle de la stabilité du complexe chimiodétecteur-cation éteint CB-Co. Elles permettent de s'assurer que l'augmentation de la fluorescence observée est bien liée à l'action de l'ion phosphate (Pi) i sur le cation Co, et non pas à l'action de chélation de l'EDTA sur le complexe chimiodétecteur-cation

éteint CB-Co.

Les tests suivants ont été réalisés trois fois chacun (session 1, session 2, session 3) pour chaque concentration de 4-MUP. La figure 8 reprend les grandes étapes des tests réalisés. La figure 8 schématise les différentes étapes du test permettant de déterminer la fluorescence additionnelle générée par un composé fluorescent selon l'invention.

Les différentes étapes du test sont reprises ci-dessous :

(i) La mesure du plastique de chaque bandelette vide est réalisée à l'aide la tête scanner de l'instrument VIDAS® 3;

(ii) Les bandelettes sont remplies (puits XA) comme suit :

a. Section A → 1 bandelette est remplie avec 300 µl de la solution B (B1, B2 ou B3) ;
b. Section B → 3 bandelettes sont remplies avec 300 µl de la solution A (A1, A2 ou A3) ;
c. Section C → 3 bandelettes sont remplies avec 300 µl de la solution C (C1, C2 ou C3).

(iii) Les bandelettes sont chargées sur un VIDAS® 3 EP VN 1015;

(iv) La lecture du bruit de fond (BKG) est réalisée à T0 pour chaque bandelette par la même tête scanner ;

(v) L'enzyme phosphatase alcaline (PAL) est ajoutée, en excès, dans chaque cuvette (10 µl à 500 µg/ml) ;

(vi) La fluorescence de chaque cuvette est mesurée toutes le cinq minutes pendant quinze minutes ;

(vii)La valeur de fluorescence relative (RFV pour Relative Fluorescence Value) est déterminée en soustrayant la valeur du bruit de fond (T0) de la valeur de la dernière lecture à t=15 minutes.

Les résultats des différents tests réalisés trois fois, à différentes concentrations en 4-MUP (0,64 µM, 0,43µM et 0,32 µM) sont repris dans les tableaux ci-dessous :

*I. Tests durant les sessions 1, 2 et 3 pour une concentration de* 4-MUP *de 0,64 µM :*

Tableau 4a. résultats obtenus pour la solution A1 (0,64 µM 4-MUP)

| 4-MUP 0,64 µM | | | |
|---|---|---|---|
| | T0 (RFU) BKG | +AP 10 µl 500µg/ml | t 15min (RFU) |
| Barrette 1.1 | 45 | 330 | 317 |
| Barrette 1.2 | 45 | 302 | 291 |
| Barrette 1.3 | 58 | 258 | 250 |
| Barrette 2.1 | 46 | 295 | 288 |
| Barrette 2.2 | 68 | 297 | 280 |
| Barrette 2.3 | 46 | 291 | 286 |
| Barrette 3.1 | 45 | 321 | 309 |
| Barrette 3.2 | 45 | 338 | 333 |
| Barrette 3.3 | 43 | 329 | 319 |

Tableau 4b. résultats obtenus pour la solution B1 (CB-Co 6,4 µM)

| CB-Co 6,4 µM | | | |
|---|---|---|---|
| | T0 (RFU) BKG | T 30sec (RFU) | t 15min (RFU) |
| Barrette 1.1 | 68 | 68 | 67 |

(suite)

| CB-Co 6,4 µM | | | |
|---|---|---|---|
| | T0 (RFU) BKG | T 30sec (RFU) | t 15min (RFU) |
| Barrette 2.1 | 62 | 64 | 63 |
| Barrette 3.1 | 86 | 87 | 86 |

Tableau 4c. : résultats obtenus pour la solution C1 (6,4 µM CB-Co + 0,64 µM 4-MUP)

| CB-Co 6,4 µM + 4-MUP 0,64 µM | | | |
|---|---|---|---|
| | T0 (RFU) BKG | +AP 10 µl 500µg/ml | t 15min (RFU) |
| Barrette 1.1 | 109 | 387 | 516 |
| Barrette 1.2 | 107 | 381 | 513 |
| Barrette 1.3 | 110 | 386 | 523 |
| Barrette 2.1 | 97 | 335 | 526 |
| Barrette 2.2 | 95 | 333 | 525 |
| Barrette 2.3 | 95 | 324 | 477 |
| Barrette 3.1 | 99 | 364 | 579 |
| Barrette 3.2 | 96 | 378 | 529 |
| Barrette 3.3 | 94 | 369 | 527 |

Tableau 4d : Valeurs relatives de fluorescence (RFV) pour l'ensemble des tests effectués à une concentration en 4-MUP de 0,64 µM.

| | **Solution A1 (4-MUP)** | **Solution C1 (4-MUP + CB-Co)** |
|---|---|---|
| | **t 15 min - BKG** | |
| | **RFV** | **RFV** |
| Barrette 1.1 | 272 | 407 |
| Barrette 1.2 | 246 | 406 |
| Barrette 1.3 | 192 | 413 |
| Barrette 2.1 | 242 | 429 |
| Barrette 2.2 | 212 | 430 |
| Barrette 2.3 | 240 | 382 |
| Barrette 3.1 | 264 | 480 |
| Barrette 3.2 | 288 | 433 |
| Barrette 3.3 | 276 | 433 |

*II. Tests durant les sessions 1, 2 et 3 pour une concentration de* 4-MUP *de 0,43 µM* :

Tableau 5a. :résultats obtenus pour la solution A2 (0,43 μM 4-MUP)

| 4-MUP 0,43 μM | | | |
|---|---|---|---|
| | T0 (RFU) BKG | +AP 10 μl 500μg/ml | t 15min (RFU) |
| Barrette 1.1 | 52 | 218 | 211 |
| Barrette 1.2 | 44 | 191 | 184 |
| Barrette 1.3 | 44 | 192 | 184 |
| Barrette 2.1 | 50 | 224 | 213 |
| Barrette 2.2 | 50 | 228 | 217 |
| Barrette 2.3 | 57 | 211 | 200 |
| Barrette 3.1 | 51 | 227 | 224 |
| Barrette 3.2 | 55 | 222 | 219 |
| Barrette 3.3 | 52 | 231 | 229 |

Tableau 5b. résultats obtenus pour la solution B2 (CB-Co 6.4 μM)

| CB-Co 6,4 μM | | | |
|---|---|---|---|
| | T0 (RFU) BKG | T 30sec (RFU | t 15min (RFU) |
| Barrette 1.1 | 62 | 62 | 61 |
| Barrette 2.1 | 61 | 62 | 62 |
| Barrette 3.1 | 63 | 61 | 61 |

Tableau 5c. : résultats obtenus pour la solution C2 (6,4 μM CB-Co + 0,43 μM 4-MUP)

| CB-Co 6,4 μM + 4-MUP 0,43 μM | | | |
|---|---|---|---|
| | T0 (RFU) BKG | +AP 10 μl 500μg/ml | t 15min (RFU) |
| Barrette 1.1 | 76 | 273 | 282 |
| Barrette 1.2 | 79 | 260 | 270 |
| Barrette 1.3 | 70 | 264 | 275 |
| Barrette 2.1 | 86 | 312 | 350 |
| Barrette 2.2 | 84 | 316 | 359 |
| Barrette 2.3 | 79 | 286 | 328 |
| Barrette 3.1 | 78 | 338 | 368 |
| Barrette 3.2 | 83 | 344 | 361 |
| Barrette 3.3 | 82 | 332 | 362 |

Tableau 5d : Valeurs relatives de fluorescence (RFV) pour l'ensemble des tests effectués à une concentration en 4-MUP de 0,43 μM.

| | **Solution A2 (4-MUP)** | **Solution C2 (4-MUP + CB-Co)** |
|---|---|---|
| | **t 15 min - BKG** | |
| | **RFV** | **RFV** |
| Barrette 1.1 | 159 | 206 |

(suite)

| | Solution A2 (4-MUP) | Solution C2 (4-MUP + CB-Co) |
|---|---|---|
| | t 15 min - BKG | |
| | RFV | RFV |
| Barrette 1.2 | 140 | 191 |
| Barrette 1.3 | 140 | 205 |
| Barrette 2.1 | 163 | 264 |
| Barrette 2.2 | 167 | 275 |
| Barrette 2.3 | 143 | 249 |
| Barrette 3.1 | 173 | 290 |
| Barrette 3.2 | 164 | 278 |
| Barrette 3.3 | 177 | 280 |

*III. Tests durant les sessions 1, 2 et 3 pour une concentration de 4-MUP de 0,32 μM* :

Tableau 5a. :résultats obtenus pour la solution A3 (0,32 μM 4-MUP)

| 4-MUP 0,32 μM | | | |
|---|---|---|---|
| | T0 (RFU) BKG | +AP 10 μl 500μg/ml | t 15min (RFU) |
| Barrette 1.1 | 50 | 138 | 134 |
| Barrette 1.2 | 60 | 146 | 142 |
| Barrette 1.3 | 52 | 139 | 134 |
| Barrette 2.1 | 42 | 132 | 129 |
| Barrette 2.2 | 48 | 130 | 127 |
| Barrette 2.3 | 46 | 137 | 135 |
| Barrette 3.1 | 42 | 123 | 121 |
| Barrette 3.2 | 47 | 128 | 126 |
| Barrette 3.3 | 44 | 123 | 122 |

Tableau 5b. résultats obtenus pour la solution B3 (CB-Co 6.4 μM) :

| CB-Co 6,4 μM | | | |
|---|---|---|---|
| | T0 (RFU) BKG | T 30sec (RFU) | t 15min (RFU) |
| Barrette 1.1 | 56 | 55 | 55 |
| Barrette 2.1 | 65 | 65 | 64 |
| Barrette 3.1 | 73 | 72 | 71 |

Tableau 5c. : résultats obtenus pour la solution C3 (6,4 μM CB-Co + 0,32 μM 4-MUP) :

| CB-Co 6,4 μM + 4-MUP 0,32 μM | | | |
|---|---|---|---|
| | T0 (RFU) BKG | +AP 10 μl 500μg/ml | t 15min (RFU) |
| Barrette 1.1 | 66 | 165 | 179 |

(suite)

| CB-Co 6,4 µM + 4-MUP 0,32 µM | | | |
|---|---|---|---|
| | T0 (RFU) BKG | +AP 10 µl 500µg/ml | t 15min (RFU) |
| Barrette 1.2 | 72 | 169 | 183 |
| Barrette 1.3 | 65 | 161 | 175 |
| Barrette 2.1 | 61 | 184 | 191 |
| Barrette 2.2 | 66 | 191 | 200 |
| Barrette 2.3 | 67 | 194 | 203 |
| Barrette 3.1 | 67 | 167 | 178 |
| Barrette 3.2 | 64 | 167 | 180 |
| Barrette 3.3 | 68 | 167 | 180 |

Tableau 5d : Valeurs relatives de fluorescence (RFV) pour l'ensemble des tests effectués à une concentration en 4-MUP de 0,32 µM.

| | **Solution A3 (4-MUP)** | **Solution C3 (4-MUP + CB-Co)** |
|---|---|---|
| | **t 15 min - BKG** | |
| | **RFV** | **RFV** |
| Barrette 1.1 | 84 | 113 |
| Barrette 1.2 | 82 | 111 |
| Barrette 1.3 | 82 | 110 |
| Barrette 2.1 | 87 | 130 |
| Barrette 2.2 | 79 | 134 |
| Barrette 2.3 | 89 | 136 |
| Barrette 3.1 | 79 | 111 |
| Barrette 3.2 | 79 | 116 |
| Barrette 3.3 | 78 | 112 |

Suite à ces différentes expériences, les valeurs moyennes RFV ont été calculées pour chaque concentration de 4-MUP, pour chaque solution A1, A2, A3 et C1, C2 et C3.

Ces valeur moyennes sont reprises dans le graphique figurant à la figure 10.

Comme il ressort de la figure 10, l'augmentation de la fluorescence moyenne entre les solutions A (A1, A2, A3) et C (C1, C2, C3) est d'environ 50%. Cette augmentation de la fluorescence est due au signal généré par le bleu de calcéine.

Exemple 5 : Test préliminaire de stabilité :

La stabilité du complexe CB-Co, du substrat enzymatique fluorogène 4-MUP et de leur mélange a été évaluée en suivant leur fluorescence dans le temps. Ceci afin de vérifier que l'hydrolyse spontanée du substrat enzymatique fluorogène 4-MUP est évitée, de même que le déplacement du cation $Co^{2+}$ du bleu de calcéine (CB).

Les résultats obtenus par la Demanderesse ont permis de démontrer qu'il n'y a pas d'interaction spontanée et que la fluorescence reste stable dans le temps (notamment entre 0 et 25 minutes)

Exemple 6 : Utilisation du complexe N-[2-(1H-benzo[d]imidazol-2-yl)phényl]-N-[(E)-1-(1H-pyrrol-2-yl)méthylidène]amine - ion cuivre ($Cu^{2+}$) à titre de complexe chimiodétecteur-cation éteint

Le complexe N-[2-(1H-benzo[d]imidazol-2-yl)phényl]-N-[(E)-1-(1H-pyrrol-2-yl)méthylidène]amine associée à

l'ion cuivre ($Cu^{2+}$), de formule (II) suivante :

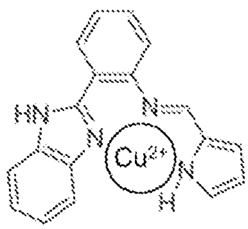

(II) ;

est un autre exemple de complexe chimiodétecteur-cation réagissant spécifiquement avec le phosphate inorganique Pi, pouvant être utilisé comme substrat secondaire.

1. <u>Synthèse de la N-[2-(1H-benzo[d]imidazol-2-yl)phényl]-N-[(E)-1-(1H-pyrrol-2-yl)-méthylidène]amine :</u>

Le composé N-[2-(1H-benzo[d]imidazol-2-yl)phényl]-N-[(E)-1-(1H-pyrrol-2-yl)méthylidène] amine est obtenu selon la réaction suivante :

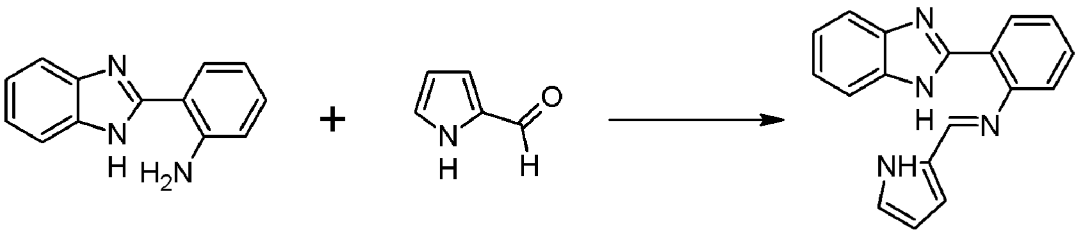

Une solution de 2-(2-aminophényl)-1H-benzimidazole (209 mg, 1,0 mmol) est mis à réagir avec du pyrrole-2-carboxaldéhyde (142 mg, 1,5 mmol) dans 50 ml de méthanol sec, et chauffée à reflux pendant 10 heures. Après réaction, le solvant est évaporé à 25 ml et maintenu à 50°C pour une évaporation lente. Le précipité blanc obtenu est ensuite filtré et lavé avec du méthanol froid à trois reprises.

2. <u>Synthèse du complexe chimiodétecteur-cation :</u>

Le complexe N-[2-(1H-benzo[d]imidazol-2-yl)phényl]-N-[(E)-1-(1H-pyrrol-2-yl)-méthylidène]amine associée à l'ion cuivre ($Cu^{2+}$), de formule (II) suivante :

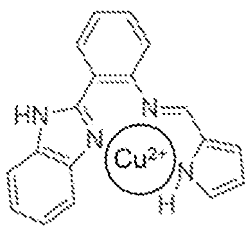

(II) ;

est obtenu en faisant réagir la N-[2-(1H-benzo[d]imidazol-2-yl)phényl]-N-[(E)-1-(1H-pyrrol-2-yl)-méthylidène]amine (286 mg, 1,0 mmol) avec du Cu(NO3)2.6$H_2O$ (295 mg, 1.0 mmol) dans du THF/$H_2O$ (30 mL, 1/1, v/v). Le mélange est chauffé à reflux pendant 8 heures. Au terme de la réaction, le produit est séparé par diffusion lente d'éther diéthylique au mélange réactionnel. Le solide obtenu est lavé au méthanol froid et un produit de couleur vert foncé est obtenu.

3. Activation du complexe chimiodétecteur-cation éteint :

Comme cela est illustré à la figure 7, ce complexe chimiodétecteur-cation éteint est activé par le phosphate inorganique Pi et permet de former d'une part un produit non-fluorescent associant le cation $Cu^{2+}$ à l'ion hydrogénophosphate (Pi-Cu) et d'autre part de libérer le composé fluorescent [2-(1H-benzo[d]imidazol-2-yl)phényl]-N-[(E)-1-(1H-pyrrol-2-yl)méthylidène]amine. La figure 11 quant à elle compare le spectre d'émission d'une composition comprenant le complexe chimiodétecteur-cation éteint (N-[2-(1H-benzo[d]imidazol-2-yl)phényl]-N-[(E)-1-(1H-pyrrol-2-yl)méthylidène]amine - ion cuivre ($Cu^{2+}$)) sans phosphate inorganique Pi avec le spectre d'émission d'une composition comprenant à la fois le complexe chimiodétecteur-cation éteint (N-[2-(1H-benzo[d]imidazol-2-yl)phényl]-N-[(E)-1-(1H-pyrrol-2-yl)méthylidène]amine - ion cuivre ($Cu^{2+}$)) et le phosphate inorganique Pi. Comme il ressort de cette figure 11, la présence du phosphate organique permet la formation d'une part du produit non-fluorescent associant le cation $Cu^{2+}$ à l'ion hydrogénophosphate (Pi-Cu), et d'autre part de libérer le composé fluorescent [2-(1H-benzo[d]imidazol-2-yl)phényl]-N-[(E)-1-(1H-pyrrol-2-yl)méthylidène]amine.

Le composé fluorescent ainsi obtenu :

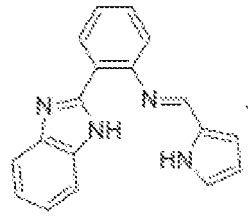

exposé ou excité par une source lumineuse de longueur d'onde d'environ 370 ± 5 nm émet des rayons lumineux ou signaux de fluorescence selon une deuxième longueur d'onde d'environ 425 ± 20 nm.

Ainsi, le produit fluorescent est excité et émet à des longueurs d'ondes sensiblement compatibles que la 4-methylumbelliferone (4-MU).

Ce complexe chimiodétecteur-cation éteint est également compatible avec le pH du VIDAS XA qui est d'environ 9,2.

Références bibliographiques

- Alvaro M. et al., 2001, Chem. Phys. Lett., 350 : 240-246
- Henary M. M. et al., 2004, Chem. Eur. J., 10: 3015-3025
- Jung H.S. et al., 2009, J. Am. Chem. Soc., 131 : 2008-2012
- Leong W. L. et Vittal J. J., 2007, Cryst. Growth Des, 7(10) : 2112-2116
- Rassasie M.J. et al., 1992, Steroids, 57: 112
- Saluja P. et al., 2012, Tetrahedron Lett., 53: 3292-3295
- Stabler T.V., et al., 1991, Clin. Chem., 37(11): 1987
- Thomas F. et al., 1999, J. Biol. Chem., 274 : 13375-13383
- Yao J. et al, 2009, Inorg. Chem Commun., 12 : 116-118
- Zhang G. et al., 2012, Sensor Actuat. B-Chem., 171- 172 : 786- 794


**Revendications**

1. Composition pour dosage immuno-enzymatique par immunofluorescence comprenant (i) un substrat enzymatique fluorogène et (ii) un composé fluorogène éteint formant un composé fluorescent après hydrolyse du substrat enzymatique fluorogène (i).

2. Composition selon la revendication 1, **caractérisée en ce que** le substrat enzymatique fluorogène est choisi parmi le 4-méthylumbelliferyl phosphate (4-MUP), le 4-methylumbelliferyl galactoside (4-MUG), le 4-méthylumbelliferyl sulfate (4-MUS), la fluorescéine di-phosphate (FDP), le 6,8-difluoro-4-méthylumbelliferyl phosphate (DiFMUP), le 9H-(1,3-dichloro-9,9-diméthylacridin-2-one-7-yl) phosphate (DDAO phosphate), le 2'-[2-benzothiazole]-6'-hydroxybenzothiazole phosphate, le 2-naphthyl phosphate et le 2-umbelliferyl phosphate.

3. Composition selon la revendication 2, **caractérisée en ce que** le substrat enzymatique fluorogène est le 4-méthylumbelliferyl phosphate (4-MUP).

4. Composition selon l'une des revendications précédentes, **caractérisée en ce que** le composé fluorogène éteint est un complexe chimiodétecteur-cation éteint.

5. Composition selon la revendication 4, **caractérisé en ce que** le chimiodétecteur du complexe chimiodétecteur-cation éteint est choisi parmi les coumarines hydrophiles et les dérivés du benzimidazole, et **en ce que** le cation du complexe chimiodétecteur-cation éteint est choisi parmi le $Co^{2+}$, le $Cr^{3+}$, le $Cu^{2+}$, le $Fe^{2+}$, le $Fe^{3+}$, le $Mn^{2+}$, le $Ni^{2+}$, le $Hg^{2+}$ et le $Pb^{2+}$.

6. Composition selon la revendication 5, **caractérisée en ce que** le complexe chimiodétecteur-cation éteint est choisi parmi :

- le complexe bleu de calcéine - ion Cobalt ($Co^{2+}$) et
- le complexe N-[2-(1H-benzo[d]imidazol-2-yl)-phényl]-N-[(E)-1-(1H-pyrrol-2-yl)méthylidène]amine - ion cuivre ($Cu^{2+}$).

7. Composition selon la revendication 4, **caractérisée en ce que** le substrat enzymatique fluorogène est le 4-méthylumbelliferyl phosphate (4-MUP) et **en ce que** le complexe chimiodétecteur-ion éteint est le complexe bleu de calcéine - ion Cobalt ($Co^{2+}$).

8. Kit pour dosage immuno-enzymatique par immunofluorescence comprenant une composition selon l'une des revendications 1 à 7.

9. Automate d'immuno-analyses comprenant une composition selon l'une des revendications 1 à 7 ou un kit selon la revendication 8.

10. Utilisation d'une composition, d'un kit ou d'un automate selon l'une quelconque des revendications précédentes, pour le dosage immuno-enzymatique par immunofluorescence d'un analyte.

11. Procédé de détection et/ou quantification *in vitro* d'un analyte par dosage immuno-enzymatique par immunofluorescence d'un échantillon liquide de test susceptible de contenir l'analyte, comprenant les étapes suivantes de :

- mise en présence d'un partenaire de capture, préalablement fixé ou non fixé sur une surface solide, et dudit échantillon liquide pour la fixation de l'analyte sur le partenaire de capture ;
- ajout d'un partenaire de détection, lequel est couplé directement ou indirectement à une enzyme capable de lyser le substrat enzymatique fluorogène d'une composition selon l'une des revendications 1 à 7, pour la fixation sur le complexe partenaire de capture-analyte ;
- mise en présence d'une composition selon l'une des revendications 1 à 7 et du complexe partenaire de capture-analyte-partenaire de détection pour la formation d'un milieu réactionnel ; et
- détection par immunofluorescence de la présence et/ou la quantité d'analyte en mesurant la fluorescence émise dans le milieu réactionnel.

12. Procédé de détection et/ou quantification *in vitro* d'un analyte selon la revendication 11, comprenant au moins l'une des étapes suivantes :

avant l'ajout du partenaire de détection, une étape de rinçage de façon à éliminer l'analyte non-lié au complexe partenaire de capture-analyte ; et
après l'ajout du partenaire de détection, une étape de rinçage de façon à éliminer le partenaire de détection non lié.

13. Procédé de détection et/ou quantification *in vitro* d'un analyte par dosage immuno-enzymatique par immunofluorescence d'un échantillon liquide de test susceptible de contenir l'analyte, comprenant les étapes suivantes de :

- mise en présence d'un partenaire de capture, préalablement fixé ou non fixé sur une surface solide, d'un analogue à l'analyte couplé à une enzyme capable de lyser le substrat enzymatique fluorogène d'une composition selon l'une des revendications 1 à 7, et dudit échantillon liquide, lesquels entrent en compétition pour la

fixation sur le partenaire de capture ;
- mise en présence d'une composition selon l'une des revendications 1 à 7 et des complexes partenaires de capture-analyte et partenaire de capture-analogue à l'analyte pour la formation d'un milieu réactionnel ; et
- détection par immunofluorescence de la présence et/ou quantité d'analyte en mesurant la fluorescence émise dans le milieu réactionnel.

**14.** Procédé de détection et/ou quantification *in vitro* d'un analyte selon la revendication 13, comprenant au moins l'une des étapes suivantes :

avant l'ajout de l'échantillon, une étape de rinçage de façon à éliminer l'analyte non-lié au complexe partenaire de capture-analyte ; et
après l'ajout de l'échantillon, une étape de rinçage de façon à éliminer l'analyte et analogue à l'analyte non lié.

**15.** Procédé d'amélioration de la sensibilité d'un procédé de détection et/ou quantification *in vitro* par dosage immunoenzymatique par immunofluorescence d'un analyte à détecter dans un échantillon de test, **caractérisé en ce qu'**il comprend l'utilisation, lors de la mise en oeuvre du dosage, d'une composition selon l'une quelconque des revendications 1 à 7 ou d'un kit selon la revendication 8.

## Patentansprüche

**1.** Zusammensetzung für einen Enzymimmunassay mittels Immunfluoreszenz, umfassend (i) ein fluorogenes Enzymsubstrat und (ii) eine gequenchte fluorogene Verbindung, die nach Hydrolyse des fluorogenen Enzymsubstrats (i) eine fluoreszierende Verbindung bildet.

**2.** Zusammensetzung nach Anspruch 1, **dadurch gekennzeichnet, dass** das fluorogene Enzymsubstrat aus 4-Methylumbelliferylphosphat (4-MUP), 4-Methylumbelliferylgalactosid (4-MUG), 4-Methylumbelliferylsulfat (4-MUS), Fluoresceindiphosphat (FDP), 6,8-Difluor-4-methylumbelliferylphosphat (DiFMUP), 9H-(1,3-Dichlor-9,9-dimethylacridin-2-on-7-yl)phosphat (DDAO-Phosphat), 2'-[2-Benzothiazol]-6'-hydroxybenzothiazolphosphat, 2-Naphthylphosphat und 2-Umbelliferylphosphat ausgewählt ist.

**3.** Zusammensetzung nach Anspruch 2, **dadurch gekennzeichnet, dass** das fluorogene Enzymsubstrat 4-Methylumbelliferylphosphat (4-MUP) ist.

**4.** Zusammensetzung nach einem der vorhergehenden Ansprüche, **dadurch gekennzeichnet, dass** die gequenchte fluorogene Verbindung ein gequenchter Chemosensor-Kation-Komplex ist.

**5.** Zusammensetzung nach Anspruch 4, **dadurch gekennzeichnet, dass** der Chemosensor des gequenchten Chemosensor-Kation-Komplexes aus hydrophilen Cumarinen und Benzimidazolderivaten ausgewählt ist und dass das Kation des gequenchten Chemosensor-Kation-Komplexes aus $Co^{2+}$, $Cr^{3+}$ , $Cu^{2+}$, $Fe^{2+}$, $Fe^{3+}$, $Mn^{2+}$, $Ni^{2+}$, $Hg^{2+}$ und $Pb^{2+}$ ausgewählt ist.

**6.** Zusammensetzung nach Anspruch 5, **dadurch gekennzeichnet, dass** der gequenchte Chemosensor-Kation-Komplex aus den Folgenden ausgewählt ist:

- dem Calceinblau-Kobaltion($Co^{2+}$)-Komplex und
- dem N-[2-(1H-Benzo[d]imidazol-2-yl)-phenyl]-N-[(E)-1-(1H-pyrrol-2-yl)methyliden]amin-Kupferion($Cu^{2+}$)-Komplex.

**7.** Zusammensetzung nach Anspruch 4, **dadurch gekennzeichnet, dass** das fluorogene Enzymsubstrat 4-Methylumbelliferylphosphat (4-MUP) ist und dass der gequenchte Chemosensor-Ion-Komplex der Calceinblau-Kobaltion($Co^{2+}$)-Komplex ist.

**8.** Kit für einen Enzymimmunassay mittels Immunfluoreszenz, das eine Zusammensetzung nach einem der Ansprüche 1 bis 7 umfasst.

**9.** Immunanalyseautomat, der eine Zusammensetzung nach einem der Ansprüche 1 bis 7 oder ein Kit nach Anspruch 8 umfasst.

**10.** Verwendung einer Zusammensetzung, eines Kits oder eines Automaten nach einem der vorhergehenden Ansprüche für einen Enzymimmunassay mittels Immunfluoreszenz eines Analyten.

**11.** Verfahren zum *in vitro*-Nachweisen und/oder -Quantifizieren eines Analyten durch einen Enzymimmunassay mittels Immunfluoreszenz einer flüssigen Testprobe, die den Analyten enthalten kann, welches die folgenden Schritte umfasst:

- Zusammenbringen eines Einfangpartners, der zuvor an eine feste Oberfläche gebunden wurde oder nicht, und der flüssigen Probe, um den Analyten an den Einfangpartner zu binden;
- Zugabe eines Nachweispartners, der direkt oder indirekt an ein Enzym gekuppelt ist, das in der Lage ist, das fluorogene Enzymsubstrat einer Zusammensetzung nach einem der Ansprüche 1 bis 7 zu lysieren, für die Bindung an den Einfangpartner-Analyt-Komplex;
- Zusammenbringen einer Zusammensetzung nach einem der Ansprüche 1 bis 7 und des Einfangpartner-Analyt-Nachweispartner-Komplexes zur Bildung eines Reaktionsmediums; und
- Nachweisen des Vorhandenseins und/oder der Menge an Analyt mittels Immunfluoreszenz durch Messen der in das Reaktionsmedium emittierten Fluoreszenz.

**12.** Verfahren zum *in vitro*-Nachweisen und/oder -Quantifizieren eines Analyten nach Anspruch 11, welches mindestens einen der folgenden Schritte umfasst:

vor der Zugabe des Nachweispartners einen Spülschritt, um den Analyten zu beseitigen, der nicht an den Einfangpartner-Analyt-Komplex gebunden hat; und
nach der Zugabe des Nachweispartners einen Spülschritt, um den ungebundenen Nachweispartner zu beseitigen.

**13.** Verfahren zum *in vitro*-Nachweisen und/oder -Quantifizieren eines Analyten durch einen Enzymimmunassay mittels Immunfluoreszenz einer flüssigen Testprobe, die den Analyten enthalten kann, welches die folgenden Schritte umfasst:

- Zusammenbringen eines Einfangpartners, der zuvor an eine feste Oberfläche gebunden wurde oder nicht, eines Analogons des Analyten, das an ein Enzym gekuppelt ist, das in der Lage ist, das fluorogene Enzymsubstrat einer Zusammensetzung nach einem der Ansprüche 1 bis 7 zu lysieren, und der flüssigen Probe, die um die Bindung an den Einfangpartner konkurrieren;
- Zusammenbringen einer Zusammensetzung nach einem der Ansprüche 1 bis 7 und der Einfangpartner-Analytsowie der Einfangpartner-Analytanalogon-Komplexe zur Bildung eines Reaktionsmediums; und
- Nachweisen des Vorhandenseins und/oder der Menge an Analyt mittels Immunfluoreszenz durch Messen der in das Reaktionsmedium emittierten Fluoreszenz.

**14.** Verfahren zum *in vitro*-Nachweisen und/oder -Quantifizieren eines Analyten nach Anspruch 13, das mindestens einen der folgenden Schritte umfasst:

vor der Zugabe der Probe einen Spülschritt, um den Analyten zu beseitigen, der nicht an den Einfangpartner-Analyt-Komplex gebunden hat; und
nach der Zugabe der Probe einen Spülschritt, um den ungebundenen Analyten und das ungebundene Analytanalogon zu beseitigen.

**15.** Verfahren zur Verbesserung der Empfindlichkeit eines *in vitro*-Nachweis- und/oder Quantifizierungsverfahrens durch einen Enzymimmunassay mittels Immunfluoreszenz eines in einer Testprobe nachzuweisenden Analyten, **dadurch gekennzeichnet, dass** es die Verwendung einer Zusammensetzung nach einem der Ansprüche 1 bis 7 oder eines Kits nach Anspruch 8 bei der Durchführung des Assays umfasst.

## Claims

**1.** A composition for enzyme immunoassay using immunofluorescence comprising (i) a fluorogenic enzymatic substrate and (ii) a quenched fluorogenic compound forming a fluorescent compound after hydrolysis of the fluorogenic enzymatic substrate (i).

2. The composition as claimed in claim 1, **characterized in that** the fluorogenic enzymatic substrate is chosen from 4-methylumbelliferyl phosphate (4-MUP), 4-methylumbelliferyl galactoside (4-MUG), 4-methylumbelliferyl sulfate (4-MUS), fluorescein diphosphate (FDP), 6,8-difluoro-4-methylumbelliferyl phosphate (DiFMUP), 9H-(1,3-dichloro-9,9-dimethylacridin-2-one-7-yl) phosphate (DDAO phosphate), 2'-[2-benzothiazole]-6'-hydroxybenzothiazole phosphate, 2-naphthyl phosphate and 2-umbelliferyl phosphate.

3. The composition as claimed in claim 2, **characterized in that** the fluorogenic enzymatic substrate is 4-methylumbelliferyl phosphate (4-MUP).

4. The composition as claimed in one of the preceding claims, **characterized in that** the quenched fluorogenic compound is a quenched chemosensor-cation complex.

5. The composition as claimed in claim 4, **characterized in that** the chemosensor of the quenched chemosensor-cation complex is chosen from hydrophilic coumarins and benzimidazole derivatives, and **in that** the cation of the quenched chemosensor-cation complex is chosen from Co2+, Cr3+, Cu2+, Fe2+, Fe3+, Mn2+, Ni2+, Hg2+ and Pb2+.

6. The composition as claimed in claim 5, **characterized in that** the quenched chemosensor-cation complex is chosen from:

   - the calcein blue - cobalt ion (Co2+) complex and
   - the N-[2-(1H-benzo[d]imidazol-2-yl)-phenyl]-N-[(E)-1-(1H-pyrrol-2-yl)-methylidene]amine- copper ion (Cu2+) complex.

7. The composition as claimed in claim 4, **characterized in that** the fluorogenic enzymatic substrate is 4-methylumbelliferyl phosphate (4-MUP) and **in that** the quenched chemosensor-ion complex is the calcein blue-cobalt ion (Co2+) complex.

8. A kit for enzyme immunoassay using immunofluorescence, comprising a composition as claimed in one of claims 1 to 7.

9. An automated device for immunoanalyses, comprising a composition as claimed in one of claims 1 to 7 or a kit as claimed in claim 8.

10. The use of a composition, of a kit or of an automated device as claimed in any one of the preceding claims, for the enzyme immunoassay using immunofluorescence of an analyte.

11. A method for in vitro detection and/or quantification of an analyte by enzyme immunoassay using immunofluorescence of a liquid test sample liable to contain the analyte, comprising the following steps of:

   - bringing together a capture partner, attached or not attached beforehand to a solid surface, and said liquid sample for binding the analyte to the capture partner;
   - adding a detection partner, which is coupled directly or indirectly to an enzyme capable of lysing the fluorogenic enzymatic substrate of a composition as claimed in one of claims 1 to 7, for binding to the capture partner-analyte complex;
   - bringing together a composition as claimed in one of claims 1 to 7 and the capture partner-analyte-detection partner complex to form a reaction medium; and
   - detecting, by immunofluorescence, the presence and/or the amount of analyte by measuring the fluorescence emitted in the reaction medium.

12. The method for in vitro detection and/or quantification of an analyte as claimed in claim 11, comprising at least one of the following steps:

   before adding the detection partner, a step of rinsing so as to eliminate the analyte not bound to the capture partner-analyte complex; and
   after adding the detection partner, a step of rinsing so as to eliminate the unbound detection partner.

13. A method for in vitro detection and/or quantification of an analyte by enzyme immunoassay using immunofluorescence of a liquid test sample liable to contain the analyte, comprising the following steps of:

- bringing together a capture partner, attached or not attached beforehand to a solid surface, an analog of the analyte coupled to an enzyme able to lyse the fluorogenic enzymatic substrate of a composition as claimed in one of claims 1 to 7, and said liquid sample, which compete to bind to the capture partner;
- bringing together a composition as claimed in one of claims 1 to 7 and the capture partner-analyte and capture partner-analyte analog complexes to form a reaction medium; and
- detecting, by immunofluorescence, the presence and/or amount of analyte by measuring the fluorescence emitted in the reaction medium.

**14.** The method for in vitro detection and/or quantification of an analyte as claimed in claim 13, comprising at least one of the following steps:

before adding the sample, a step of rinsing so as to eliminate the analyte not bound to the capture partner-analyte complex; and
after adding the sample, a step of rinsing so as to eliminate the unbound analyte and analyte analog.

**15.** A method for improving the sensitivity of a method for in vitro detection and/or quantification, by enzyme immunoassay using immunofluorescence, of an analyte to be detected in a test sample, **characterized in that** it comprises the use of a composition as claimed in one of claims 1 to 7 or a kit as claimed in claim 8 while carrying out the assay.

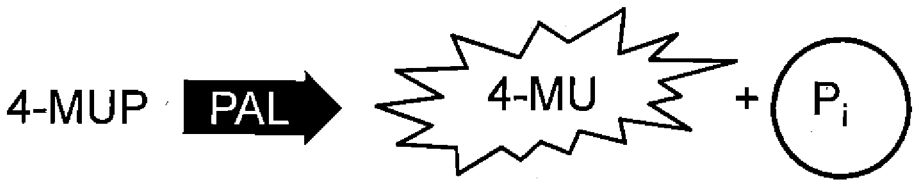


FIG. 1

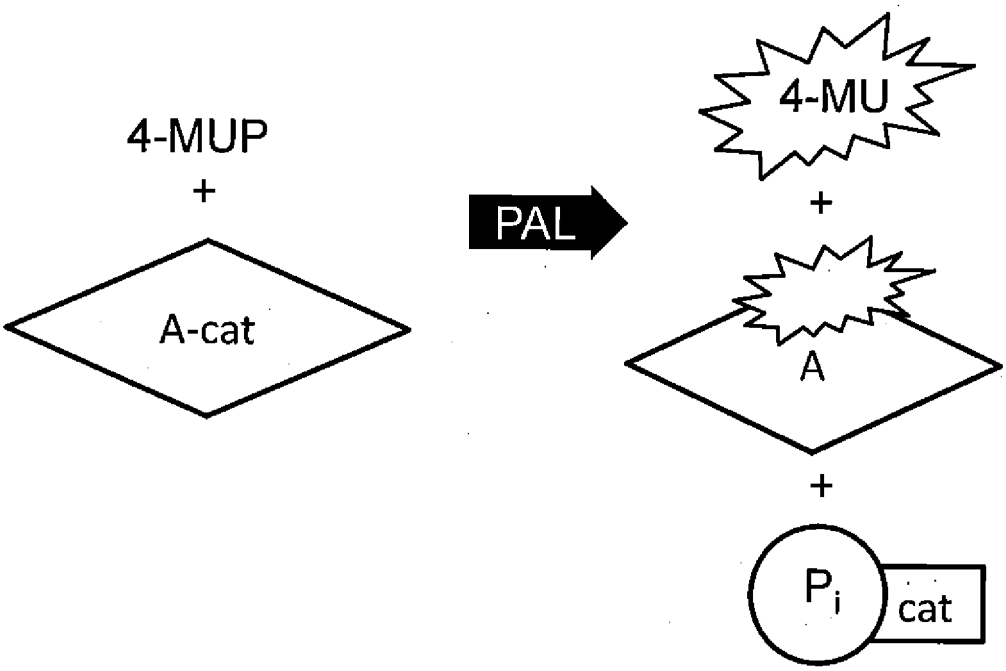


FIG. 2

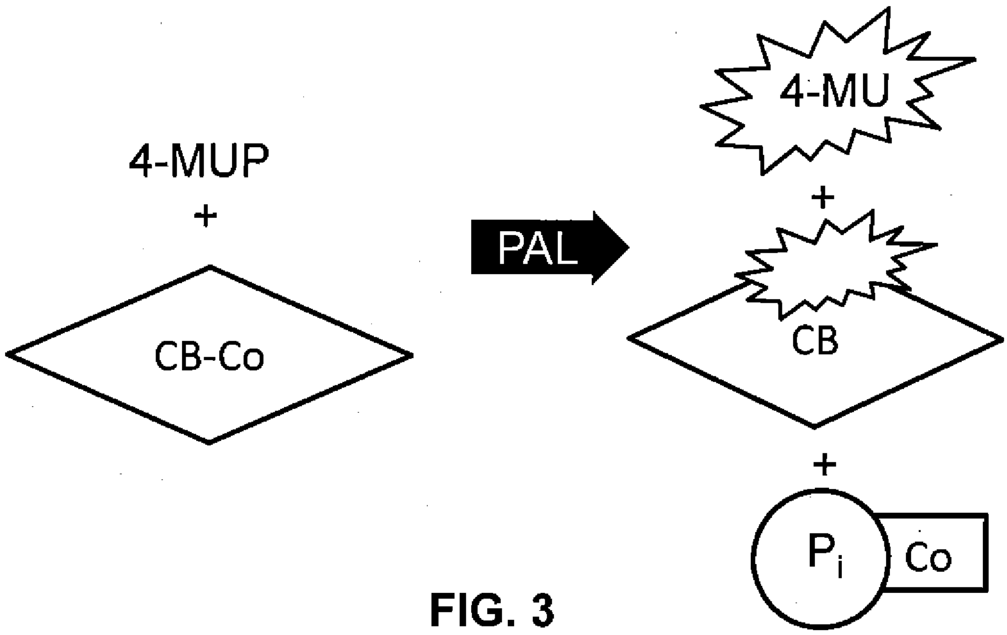
4-MUP
+
CB-Co
PAL
4-MU
+
CB
+
Pi
Co

FIG. 3

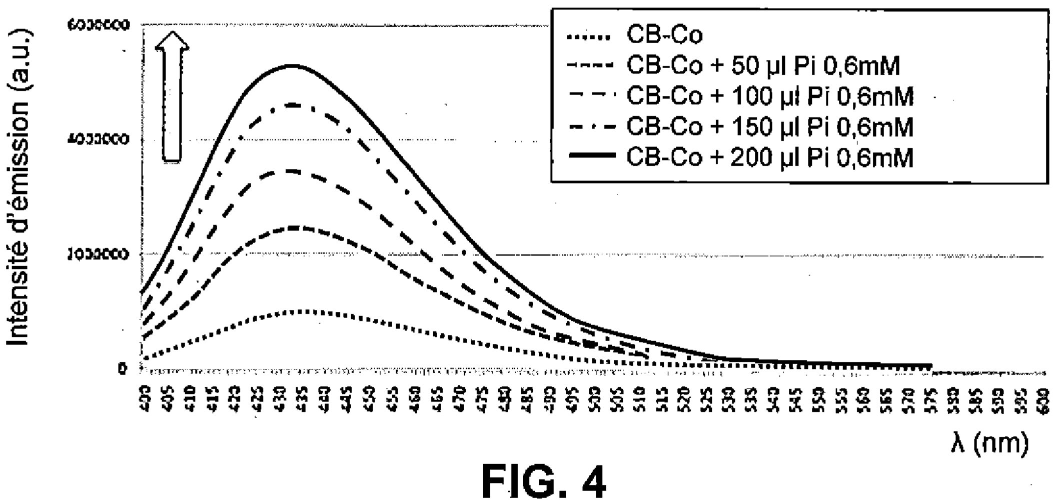
Intensité d'émission (a.u.)
CB-Co
CB-Co + 50 µl Pi 0,6mM
CB-Co + 100 µl Pi 0,6mM
CB-Co + 150 µl Pi 0,6mM
CB-Co + 200 µl Pi 0,6mM
λ (nm)

FIG. 4

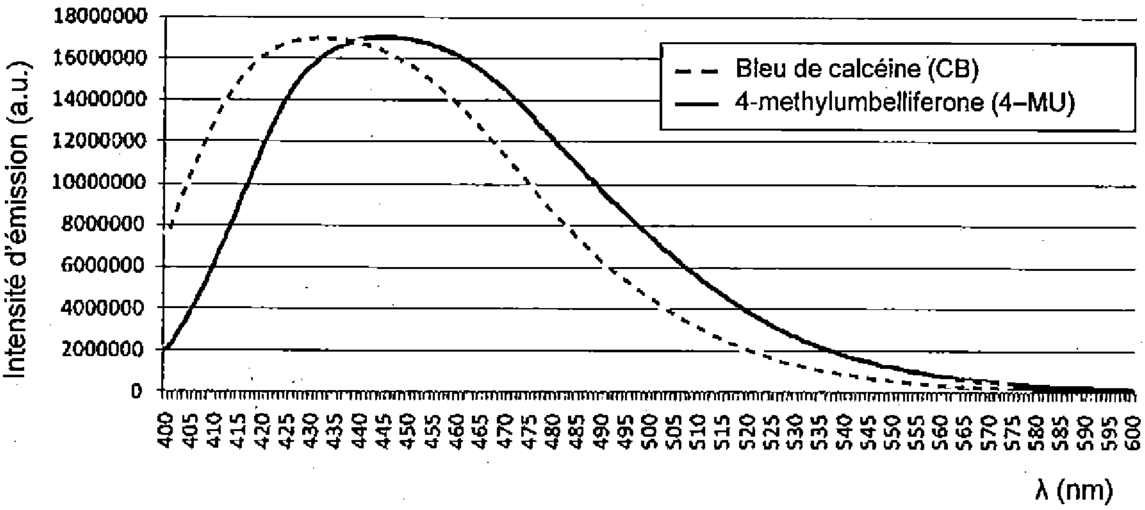
Intensité d'émission (a.u.)
18000000
16000000
14000000
12000000
10000000
8000000
6000000
4000000
2000000
0
Bleu de calcéine (CB)
4-methylumbelliferone (4-MU)
λ (nm)

FIG. 5

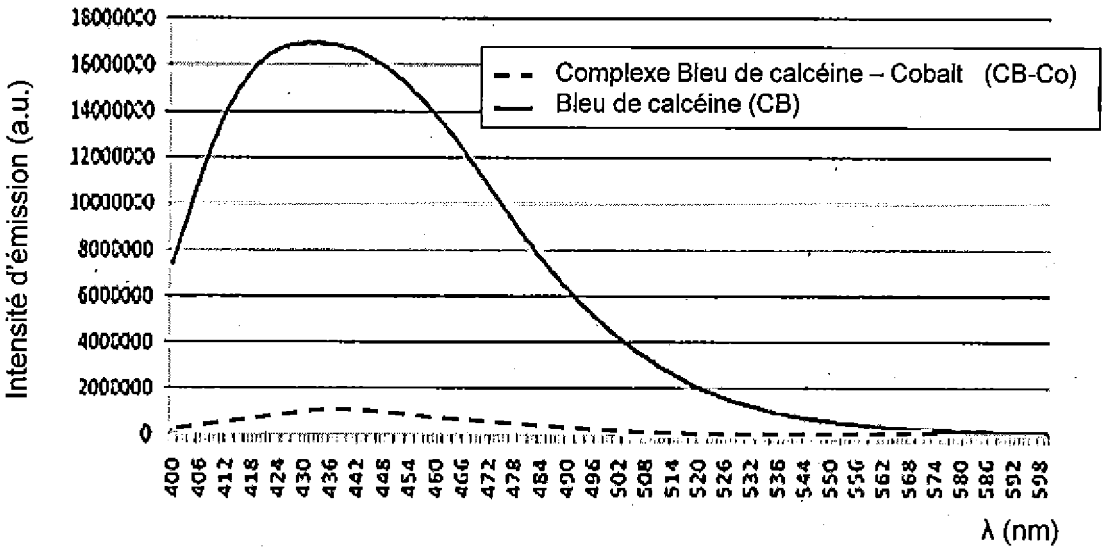
Intensité d'émission (a.u.)
18000000
16000000
14000000
12000000
10000000
8000000
6000000
4000000
2000000
0
Complexe Bleu de calcéine – Cobalt (CB-Co)
Bleu de calcéine (CB)
λ (nm)

FIG. 6

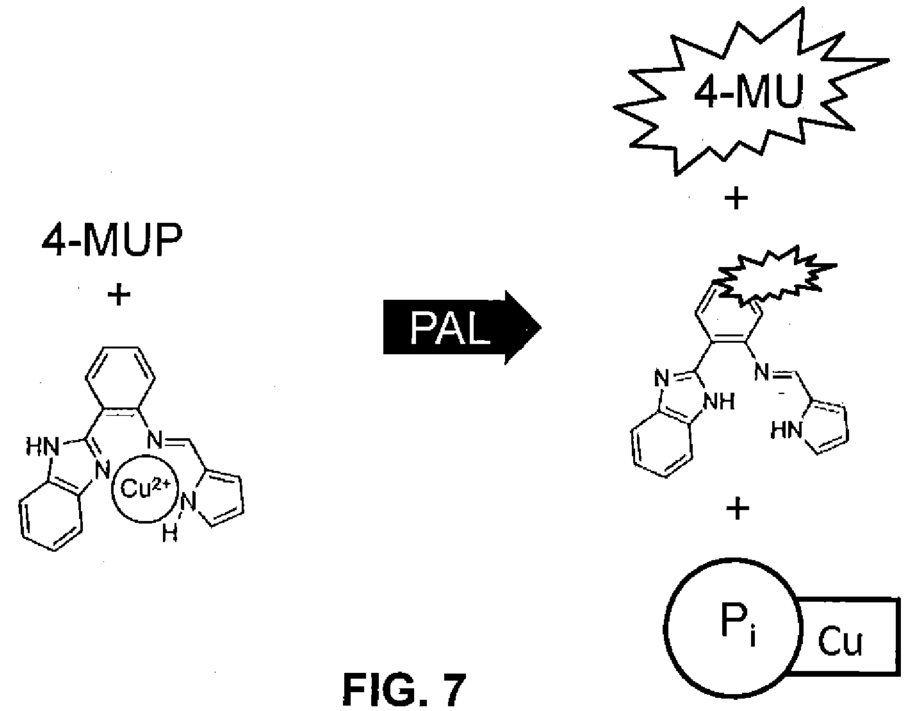
4-MUP
PAL
4-MU

FIG. 7

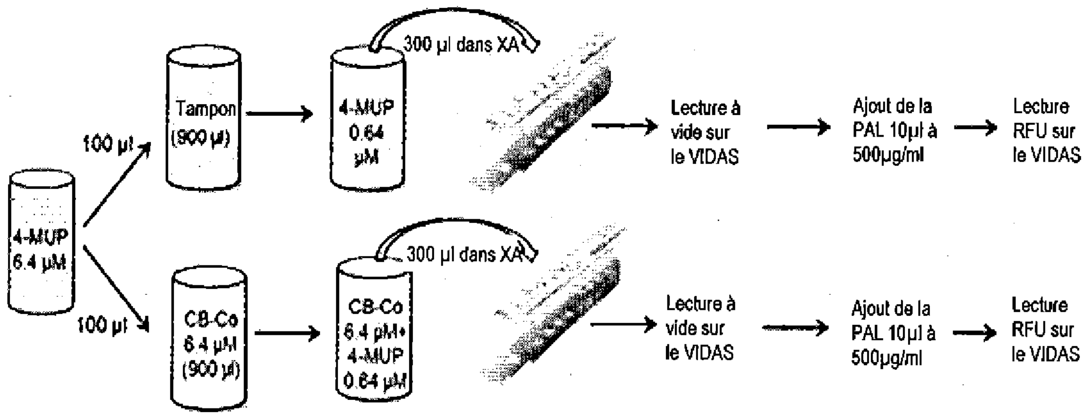
4-MUP
6.4 µM
100 µl
Tampon
(900 µl)
4-MUP
0.64
µM
300 µl dans XA
Lecture à
vide sur
le VIDAS
Ajout de la
PAL 10µl à
500µg/ml
Lecture
RFU sur
le VIDAS
100 µl
CB-Co
6.4 µM
(900 µl)
CB-Co
6.4 µM+
4-MUP
0.64 µM
300 µl dans XA
Lecture à
vide sur
le VIDAS
Ajout de la
PAL 10µl à
500µg/ml
Lecture
RFU sur
le VIDAS

FIG. 8

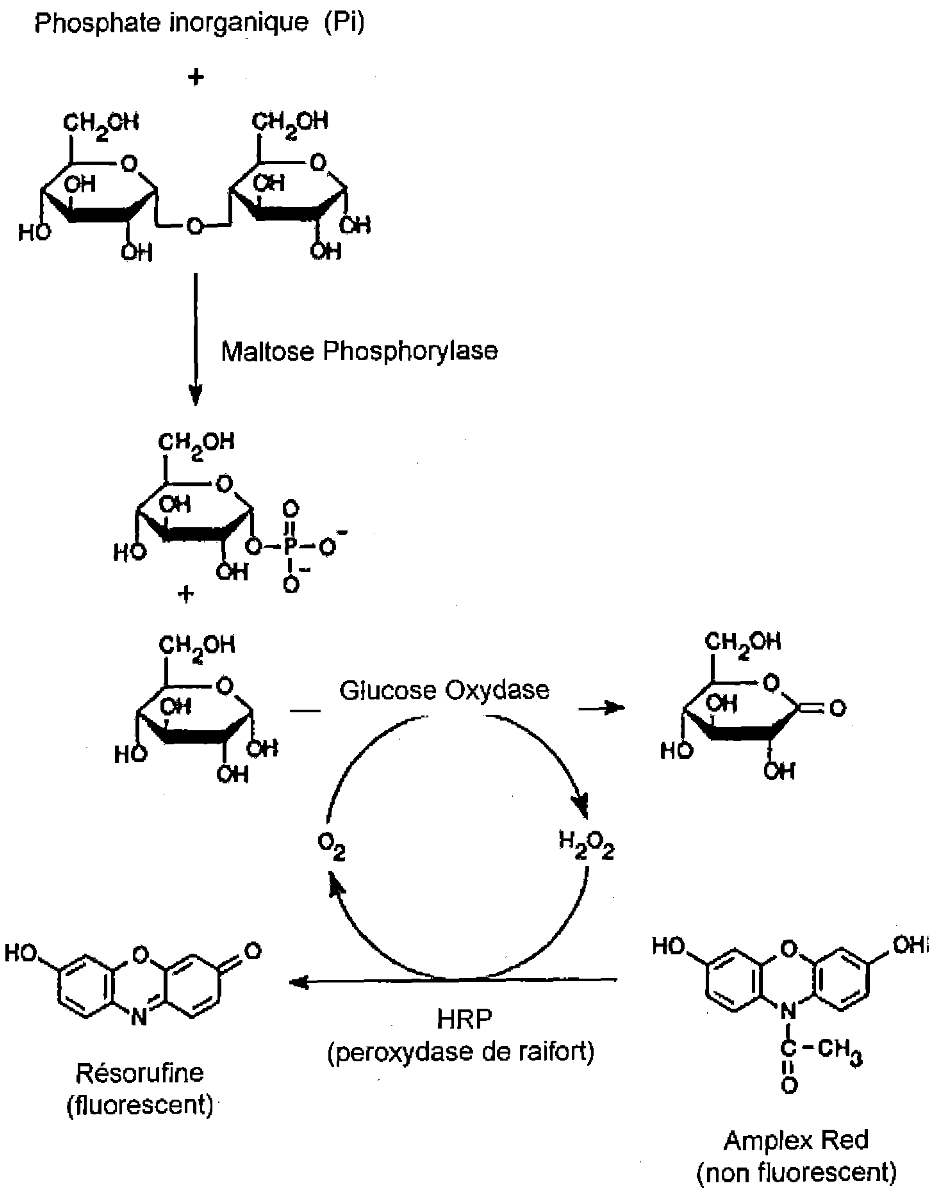
Phosphate inorganique (Pi)
+
Maltose Phosphorylase
+
Glucose Oxydase
HRP
(peroxydase de raifort)
Résorufine
(fluorescent)
Amplex Red
(non fluorescent)

**FIG. 9**

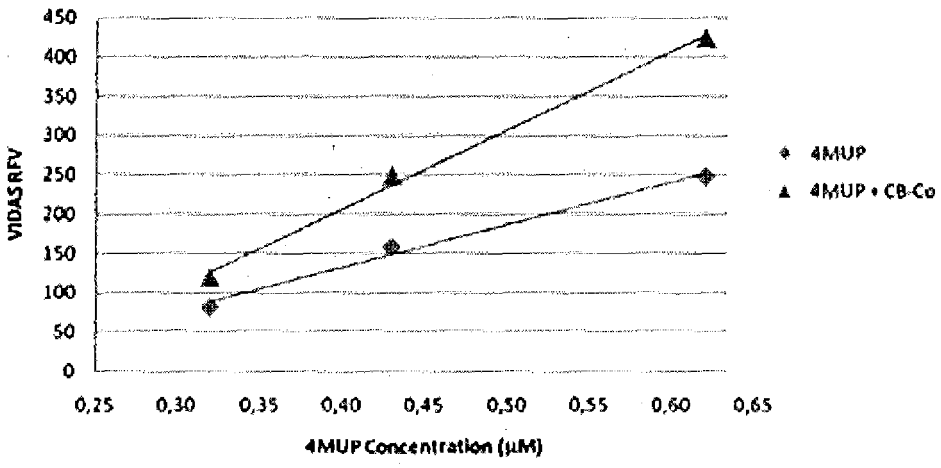
450
400
350
300
250
200
150
100
50
0
VIDAS RFV
0,25
0,30
0,35
0,40
0,45
0,50
0,55
0,60
0,65
4MUP Concentration (µM)
4MUP
4MUP + CB-Co

FIG. 10

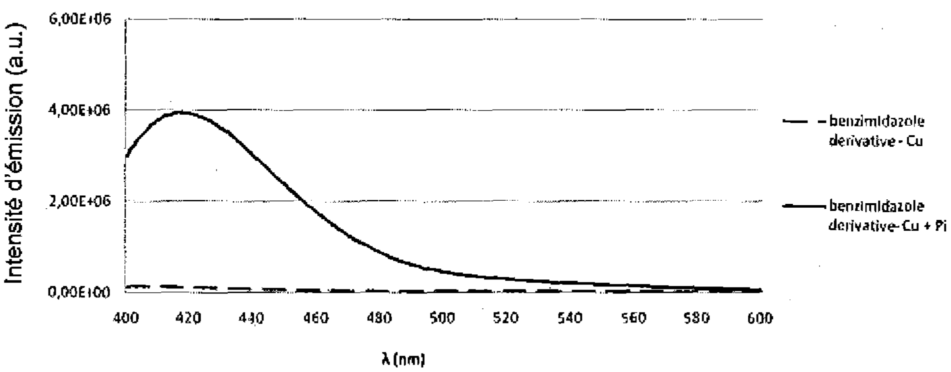
6,00E+06
4,00E+06
2,00E+06
0,00E+00
Intensité d'émission (a.u.)
400
420
440
460
480
500
520
540
560
580
600
λ (nm)
benzimidazole derivative - Cu
benzimidazole derivative-Cu + Pi

FIG. 11

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- US 3551295 A
- US 5854008 A


### Littérature non-brevet citée dans la description


- **PRETTI et al.** *Tetrahedron letters,* 2012, vol. 53, 3292-3295
- **GIBBS et al.** Selecting the Detection System - Colorimetric, Fluorescent, Luminescent Methods. *Elisa Technical Bulletin - No. 5,* 2001
- **ALVARO M. et al.** *Chem. Phys. Lett.,* 2001, vol. 350, 240-246
- **HENARY M. M. et al.** *Chem. Eur. J.,* 2004, vol. 10, 3015-3025
- **JUNG H.S. et al.** *J. Am. Chem. Soc.,* 2009, vol. 131, 2008-2012
- **LEONG W. L. ; VITTAL J. J.** *Cryst. Growth Des,* 2007, vol. 7 (10), 2112-2116
- **RASSASIE M.J. et al.** *Steroids,* 1992, vol. 57, 112
- **SALUJA P. et al.** *Tetrahedron Lett.,* 2012, vol. 53, 3292-3295
- **STABLER T.V. et al.** *Clin. Chem.,* 1987, vol. 37 (11
- **THOMAS F. et al.** *J. Biol. Chem.,* 1999, vol. 274, 13375-13383
- **YAO J. et al.** *Inorg. Chem Commun.,* 2009, vol. 12, 116-118
- **ZHANG G. et al.** *Sensor Actuat. B-Chem.,* 2012, vol. 171- 172, 786-794